# EUROPEAN PATENT APPLICATION

(11) **EP 4 052 700 A1**
(43) Date of publication of application: **07.09.2022**
(21) Application number: 21461519.7
(22) Date of filing: 03.03.2021
(51) Int. Cl.: A61K 31/00, A61K 31/506, A61K 31/55, A61P 3/04, A61P 3/10

(54) **INHIBITOR OF PROTEIN KINASE D (PKD) FOR USE IN PREVENTION OR TREATMENT OF OBESITY AND A PHARMACEUTICAL COMPOSITION FOR SUCH USE**

(71) Applicant: Instytut Biologii doswiadczalnej imienia Marcelego Nenckiego Polskiej Akademii Nauk, 02-093 Warszawa (PL); Universität Würzburg, 97080 Würzburg (DE)
(72) Inventor: SUMARA, Grzegorz, 02-972 Warszawa (PL); TRUJILLO VIERA, Jonathan, 97070 Würzburg (DE)
(74) Representative: Patpol Kancelaria Patentowa Sp. z o.o.

(57) **Abstract**

The invention relates to a PKD protein kinase inhibitor for use in preventing or treating obesity in a subject, including preferably also related conditions such as diabetes, wherein the PKD protein kinase inhibitor is administered orally, wherein the PKD protein kinase inhibitor is targeted to act locally in the digestive system, especially in the small intestine. The invention also relates to a pharmaceutical composition comprising a PKD protein kinase inhibitor for use according to the invention.

## Description

### Field of the invention

The present invention relates to a PKD protein kinase inhibitor for use in the prevention or treatment of obesity. The invention also relates to a pharmaceutical composition containing a PKD protein kinase inhibitor for use according to the invention.

### Background

The type of diet plays a major role in modulating organismal metabolism. Diets containing elevated fat content are generally more energy-dense, which promotes a positive energy balance and, consequently, obesity. The digestive system is the first place to come into contact with fats.in the diet. After emulsification of ingested fat by bile acids, triglycerides are broken down into glycerol, monoglyceride and fatty acids (FAs) by pancreatic lipases in the small intestine lumen (Hussain, 2014, Lowe, 2002). Monoglycerides and FAs are then taken up by enterocytes either by passive diffusion or by an active mechanism involving FAs transporters such us Cluster of differentiation 36 (CD36) (Hussain, 2014, Xu, Jay et al., 2013). In enterocytes, FAs and monoglycerides or glycerol are re-esterified at the endoplasmic reticulum (ER). These monoglyceride and glycerol 3-phosphate pathways are responsible for the majority of TG synthesis in enterocytes (Yang & Nickels, 2015). Following re-esterification, TG are packed into pre-chylomicrons together with lipoproteins such as apolipoprotein B48 (APOB48) and apolipoprotein A4 (APOA4) by the microsomal transfer protein (MTTP) (Mansbach & Siddiqi, 2016). APOB48 is absolutely required for pre-chylomicron formation at the ER (Mansbach & Siddiqi, 2016), while APOA4 is likely responsible for determining final chylomicron size (Kohan, Wang et al., 2012, Kohan, Wang et al., 2015, Lu, Yao et al., 2006, Weinberg, Gallagher et al., 2012). Following their assembly, pre-chylomicrons are then transported to the Golgi apparatus to undergo further chemical modifications and are designated for secretion (Hesse, Jaschke et al., 2013).

Increased dietary fat content leads to the elevation in expression and activity of enzymes critical for lipid uptake, FA re-esterification, TG packing as well as lipoproteins required for assembly of pre-chylomicrons (Clara, Schumacher et al., 2017, Hernandez Vallejo, Alqub et al., 2009, Petit, Arnould et al., 2007). Interestingly, an increase in chylomicron size might be a major factor determining the elevated capacity of enterocytes to process excessive dietary fat (Uchida, Whitsitt et al., 2012). However, the signalling cascades driving the adaptation of enterocytes to increased lipid loads in the intestinal lumen remain largely unknown.

Overconsumption of energy-dense diets is the major cause for the development of obesity. Generally, lipids are one of the most energetic components of the diet; and increased fat consumption often promotes a positive energy balance. The capacity of the digestive system to absorb lipids increases in response to the elevated fat content in the diet (Clara et al., 2017, Hernandez Vallejo et al., 2009, Petit et al., 2007). This process involves stimulation of expression and function of digestive enzymes (which degrade TG into FFAs, monoglycerides, and glycerol) and components of the enzymatic machinery responsible for FFA uptake, TG re-synthesis, packing of TG into chylomicrons, and their secretion into the lymphatic circulation (Clara et al., 2017, Hernandez Vallejo et al., 2009, Petit et al., 2007). While a reduction of fat absorption in the intestine represents an attractive strategy to mitigate weight gain, limited pharmacological strategies exist.

The PKD family includes three members (PKD1, PKD2, and PKD3) which regulate several aspects of cellular metabolism and cell pathophysiology (Fielitz, Kim et al., 2008, Ittner, Block et al., 2012, Kim, Fielitz et al., 2008, Kleger, Loebnitz et al., 2011, Kolczynska et al., 2020, Konopatskaya, Matthews et al., 2011, Löffler et al., 2018, Mayer et al., 2019, Mayer, Valdes et al., 2020, Rozengurt, 2011, Sumara et al., 2009). The present inventor's recent study suggested that PKDs might be activated in response to free fatty acids (FFAs) or DAG. Moreover, high-fat diet (HFD) feeding activated PKDs in the liver (Mayer et al., 2019). Previous studies have shown that the protein kinase D family, in particular protein kinase D1 (PKD1 or PRKD1), regulates the metabolism of beta pancreatic cells (Sumara G. et al., Cell, 2009) and the dissipation of energy in the form of heat by adipocytes. Importantly, the removal of PKD1 from adipocytes makes mice resistant to the development of obesity and type 2 diabetes (Loeffler M. et al., EMBO J., 2018). Similarly, removal of the protein kinase D3 from hepatocytes in mice makes these animals partially resistant to the development of type 2 diabetes mellitus. However, the impact of PKDs activity on lipid metabolism in the intestine has not been investigated so far.

The present inventors have showed that PKD2 is activated upon lipids loading in intestine and promotes chylomicron growth, and consequently TG secretion by human and mouse enterocytes. Interestingly, PKD2 directly phosphorylates one of the apolipoproteins associated with chylomicrons, namely APOA4. Deletion of PKD2 in intestine of mice or in human enterocytes results in increased abundance of intracellular and secreted APOA4. The activity of PKD2 in the human intestine correlates with the level of TG in the blood. At the same time, inhibition of PKD2 or specific removal of this kinase in the intestine resulted in reduced fat absorption, increased fecal energy excretion and resistance to obesity induced by a high-fat diet and resistance to diabetes or pathological changes in the intestinal microbiota. In particular, the inventors have shown that oral administration of PKD inhibitors, including PKD2, such as CRT0066101 and CID 755673, significantly reduces obesity induced by a high-fat diet, prevents the development of diabetes and improves the intestinal microflora profile of mice. The inventors found that the use of a PKD-specific inhibitor reduces fat absorption and is effective in treating obesity and related conditions in animal models.

The present inventors have also shown that inactivation of PKD2 is associated with positive changes in the intestinal microflora. Intestinal dysbiosis, defined as the loss of microbial diversity, including specific types of microorganisms, is associated with obesity, diabetes and cardiovascular disease (Henao-Mejia, Elinav et al., 2012, Qin, Li et al., 2012, Wilkins, Monga et al. al., 2019). However, the data presented herein indicate improved stability of microbial diversity in the absence of PKD2. In addition, changes were detected in bacterial taxa belonging to the genus *Bacteroides* in the duodenum. A decline has already been reported for this type in various obesity models and in response to a high-fat diet, and the results shown here indicate that deletion of PKD2 prevents adverse microflora changes (see Martinez-Guryn, Hubert et al., 2018, Turnbaugh et al., 2006) .

It should be noted here that the inhibition of PKD obtained with the low doses of the inhibitor used here resulted in inactivation only in the intestine and not in other organs. Without being bound by theory, this phenomenon may explain the fact of only a marginal increase in energy expenditure in mice treated with a PKD inhibitor.

The subject of the present invention is therefore the use of inhibition of the activity of PKD, in particular PKD2, to reduce the absorption of fats in the intestine, as well as to inhibit weight gain (in particular when eating a high-fat diet), and to improve glucose tolerance and insulin sensitivity (in particular in eating a high-fat diet). Inhibition of the activity of these kinases is achieved by oral administration of the inhibitor, wherein the inhibitory effect should be limited only or mainly to local action, in the digestive system, particularly in the intestine. Limiting the inhibition of PKD activity, in particular PKD2, to local or mainly local action, preferably only in the small intestine, allows obtaining a beneficial effect for the treatment or prevention of obesity, limiting the possibility of side effects. Limiting the action of the inhibitor only or mainly to the digestive system, in particular the intestine, can be achieved, for example, by using low doses of the inhibitor. For example, a PKD inhibitor may be administered to humans once daily at a dose in the range 0.1-20 mg/kg body weight. Preferably, the PKD inhibitor may be administered to humans once daily at a dose in the range 5-20 mg/kg body weight, more preferably, e.g. in the range 5-10 mg/kg body weight. Alternatively, with particular reference to the inhibitor CRT0066101 (a compound of Formula I): 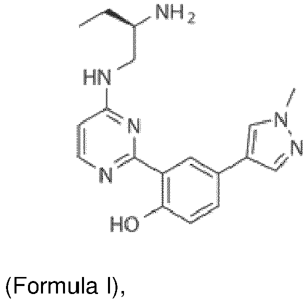 a PKD inhibitor can be administered to humans once daily in the range 0.1-5 mg/kg body weight. The appropriate dosage will depend on the patient (e.g., gender, age, medical condition, diet) and stage of obesity, and other factors will be considered by the skilled person. The appropriate dose of a PKD inhibitor that ensures no entry into the bloodstream or minimizes entry into the bloodstream can be selected by a skilled person without undue experimentation (e.g. by determination of the inhibitor level in the blood after administration), but will preferably fall within the ranges given above.

Another possibility of limiting the crossing of the intestinal barrier is the use of a chemical modification of the inhibitor that will limit or prevent its absorption in the digestive system, in particular crossing the intestinal barrier (e.g. through the appropriate electric charge of the molecule). Yet another possibility is to use a PKD inhibitor in combination with an agent or agents that limit absorption in the digestive system, in particular in the intestine (e.g. compounds that inhibit OATP2B1, e.g. food dyes, e.g. azo dyes, as well as benzalkonium chlorides, parabens, including butylparaben, inosine, surfactants and emulsifiers, e.g. SLS, and others). Another possibility is the use of a PKD inhibitor in combination with a suitable carrier, e.g. carrier particles that limit absorption in the digestive system, e.g. crossing the intestinal barrier and penetration into the bloodstream. One skilled in the art will be able to point out suitable means for targeting the inhibitor to local action in the digestive system, in particular in the intestine, in particular in the small intestine.

The unexpected finding that it is possible to obtain a beneficial effect in the treatment or prevention of obesity by targeting the inhibition of PKD activity only or mainly to the digestive system, in particular the intestine, is also associated with the possibility of obtaining reduced fat absorption and lower body weight gain on a high-fat diet using low-fat diets. inhibitor doses. For example, doses of an CRT0066101 inhibitor administered to mice in the prior art, e.g., in a cardiovascular disorder study, were e.g. 80 mg/kg body weight per day or more. In contrast, in the present invention, the effect of reducing gut fat absorption, increasing APOA4 levels, and reducing body weight gain on a high fat diet is already provided at a dose of as little as 10 mg/kg body weight per day. The effect in treating already developed obesity in mice was also seen at this inhibitor dose.

The present inventors have shown that it is essential to inhibit the activity of PKD, in particular PKD2, in order to obtain a beneficial effect in the treatment or prevention of obesity. Inhibitors that inhibit proteins of this group are known in the art.

CRT0066101 is a PKD1/2/3 specific inhibitor. It is a compound of Formula (I):

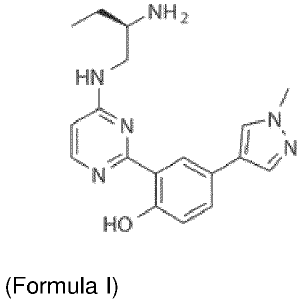

This compound is commercially available, incl. from Absource Diagnostics GmbH and Biozol. CID755673 is a PKD1, PKD2 i PKD3 inhibitor, of Formula II:

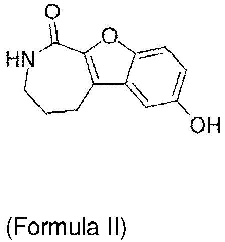

This compound is also commercially available, incl. from MedChemExpress.

Examples of other suitable inhibitors for use in the present invention are:
- pirazol[3,4-d]pyrimidine analogs, including 3-IN-PP1 (see Verschueren et al., Discovery of a potent protein kinase D inhibitor: insights in the binding mode of pyrazolo[3,4-d]pyrimidine analogue. Medchemcomm. 2017 Mar 1; 8(3): 640-646); said compound can be administered orally, e.g. preferably administered to humans once daily at a dose in the range 5-20 mg/kg body weight, e.g. 5-10 mg/kg body weight or alternatively 10-20 mg/kg body weight;
- 1-NM-PP1 (1-(tert-butyl)-3-(nafthalen-1-ylmethyl)-1H-pirazol[3,4-d]pyrimidine-4-amine) - compound of Formula III: 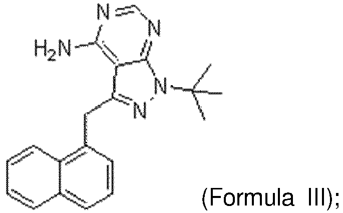 said compound can be administered orally, e.g. preferably administered to humans once daily at a dose in the range 5-20 mg/kg body weight, e.g. 5-10 mg/kg body weight or alternatively 10-20 mg/kg body weight;
- CID2011756 - compound of Formula IV: 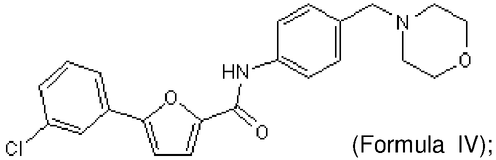 said compound can be administered orally, e.g. preferably administered to humans once daily at a dose in the range 5-20 mg/kg body weight, e.g. 5-10 mg/kg body weight or alternatively 10-20 mg/kg body weight;
- CID755673 - compound of Formula V: 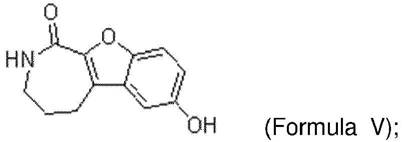 said compound can be administered orally, e.g. preferably administered to humans once daily at a dose in the range 5-20 mg/kg body weight, e.g. 5-10 mg/kg body weight or alternatively 10-20 mg/kg body weight;
- Kb NB 142-70 - compound of Formula IV: 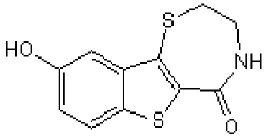 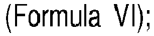 said compound can be administered orally, e.g. preferably administered to humans once daily at a dose in the range 5-20 mg/kg body weight, e.g. 5-10 mg/kg body weight or alternatively 10-20 mg/kg body weight.

Until now, there have only been some pharmacological strategies directed to limiting the absorption of fat in the intestine. Orlistat and related drugs, targeting the activity of pancreatic lipase, are currently approved for the treatment of obese patients who are refractory to lifestyle interventions (Pilitsi et al., 2019). However, these drugs have limited efficacy during prolonged treatment and their usage is often discontinued because of associated side effects such as oily stools, oily spotting, fecal urgency, fecal incontinence, hyper-defecation, and flatus with discharge (Pilitsi et al., 2019). Moreover, orlistat and related drugs do not protect from the cardiovascular complications often associated with obesity and they ameliorate the development of diabetes only to a limited extent. As presented herein, inhibition of PKDs in the intestine of mice or deletion as well as inactivation of PKD2 in animals led to similar protection from body weight gain as described previously for orlistat (Murtaza et al., 2017, Zhao et al., 2018). Importantly, while it has been found that PKD2 inactivation or inhibition of PKDs increased stool lipid content, no associated overt gastrointestinal disturbances or symptoms were observed.

It should be noted that it has already been shown that inhibition of PKD may also lead to a beneficial effect in other pathological conditions associated with obesity. Inactivation of PKD using specific inhibitors improves heart function (Venardos et al., 2015), reduces the incidence of pancreatitis (Thrower et al., 2011, Yuan et al., 2017), and prevents progression of different cancer types (Borges et al., 2015, Harikumar et al., 2010, Li et al., 2018, Sua et al., 2019). Inhibition of the PKD pathway might also lead to increased energy dissipation by adipose tissue (Löffler et al., 2018). Nevertheless, it was previously indicated that oral administration of CRT0066101, at a dose 8 times higher than that used in the experiments described here, provided the highest concentrations in pancreatic tumors after 2 hours, but the reduction in PKD1/2 activation reached 50% (Harikumar et al., 2010) . Meanwhile, the present inventors have shown that it is possible to administer the inhibitor orally, including in particular much lower doses, with an effect limited to the digestive system only.

At the same time, many data so far suggest that inhibiting PKD may cause side effects such as increased lipid accumulation and liver fibrosis, altered β-cell functions in the pancreas or increased intestinal permeability (Mayer et al., 2019, Sumara et al., 2009, Xiao, Wang et al., 2018, Xiong, Zhou et al., 2016, Zhang, Liu et al., 2020). However, the present inventors have observed that inhibition of PKD2 activity, in particular limited to only the digestive system, in particular the intestine, e.g. with relatively low doses of the inhibitor administered orally, is not associated with such side effects.

The invention therefore provides a PKD protein kinase inhibitor for use in the prevention or treatment of obesity in a subject, preferably including related conditions such as diabetes, wherein the PKD protein kinase inhibitor is administered orally, wherein the PKD protein kinase inhibitor is targeted to a local action in the digestive system, especially in the small intestine.

Preferably, the inhibitor of PKD protein kinases is a PKD2 inhibitor, in particular a specific PKD2 inhibitor.

As used herein, a PKD2 protein kinase inhibitor is a compound having an activity that inhibits the activity of protein kinases D, including in particular a compound that specifically inhibits the activity of PKD2. Examples of PKD2 protein kinase inhibitors are CRT0066101 (Formula I), CID755673 (Formula II), 3-IN-PP1, 1-NM-PP1 (Formula III), CID2011756 (Formula IV), CID755673 (Formula V), Kb NB 142-70 (Formula VI) etc.

A specific inhibitor of PKD2 is a compound, which inhibitory activity is limited to PKD2 only (without a significant effect on other PKD kinases) or a compound, which inhibitory activity towards PKD2 is significantly higher than the inhibitory activity towards other PKDs (e.g. compared to IC₅₀ values) .

By conditions associated with obesity is meant a variety of pathological conditions coexisting with obesity, such as diabetes, cardiovascular disorders, etc.

By targeting a local action in the digestive system, particularly in the small intestine, it is meant that the inhibitor is administered in such a way as to limit or even prevent intestinal barrier crossing and systemic action. This can be achieved, for example, by the use of low doses of the inhibitor, its chemical modifications or an appropriate formulation as described above.

In a preferred embodiment, the PKD protein kinase inhibitor is administered in a manner that limits intestinal barrier crossing.

In a preferred embodiment, the subject is a high fat food eater, e.g., a subject on a high fat diet.

The terms "high fat diet" or "high fat food consumption" or "fat rich diet" or "high-fat diet" or "HFD" are used interchangeably herein and mean eating meals with a minimum energy content of 30% they provide fats. By "fats" is meant nutrients from the group of lipids, in particular glycerol fatty acid esters and derivative compounds.

In a preferred embodiment, the PKD protein kinase inhibitor is a compound of Formula I 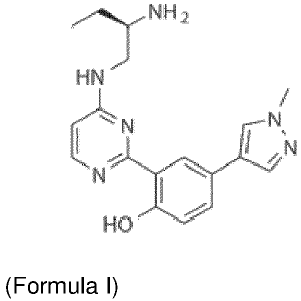 or a pharmaceutically acceptable salt thereof.

In a preferred embodiment, the subject is human.

In a preferred embodiment, the PKD protein kinase inhibitor is administered at a dose 0,1 - 20 mg/kg body weight per day.

In a preferred embodiment, the PKD protein kinase inhibitor is administered at a dose 5 - 20 mg/kg body weight per day.

In a preferred embodiment, the PKD protein kinase inhibitor is administered at a dose 5 - 10 mg/kg body weight per day.

In a preferred embodiment, the PKD protein kinase inhibitor is administered at a dose 0,1 - 5 mg/kg body weight per day.

The invention also relates to a pharmaceutical composition containing a PKD protein kinase inhibitor and pharmaceutically acceptable excipients or auxiliary ingredients for use in the invention.

In a preferred embodiment, the composition comprises at least one auxiliary ingredient to reduce absorption in the digestive system, in particular in the intestine.

The methods of formulating suitable pharmaceutical compositions are known in the art and described in the Polish Pharmacopoeia or the European Pharmacopoeia. Oral compositions generally comprise an inert diluent or an edible carrier. For oral therapeutic administration, the active compound can be combined with excipients and administered in the form of tablets, troches or capsules, e.g. gelatin capsules. The tablets, pills, capsules, troches and the like may contain any of the following ingredients, or compounds of a similar nature: a binder such as microcrystalline cellulose, tragacanth gum or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin, or a flavoring agent such as peppermint, methyl salicylate or orange flavor.

Oral compositions can also be prepared using a liquid carrier for the preparation of solutions, suspensions, or dispersions. The compositions may also be prepared for use as a mouthwash. Pharmaceutically acceptable binders and/or adjuvant materials can be included as part of the composition. Exemplary oral solutions or suspensions may include the following ingredients: water, saline, fixed oils, polyethylene glycols, glycerin, propylene glycol, or other synthetic solvents; antibacterial agents such as benzyl alcohol or methyl parabens; antioxidants such as ascorbic acid or sodium bisulfite; chelating compounds such as edetic acid; buffers such as acetates, citrates, or phosphates; and tonicity adjusting agents such as sodium chloride or dextrose. The pH can be adjusted with acids or bases such as hydrochloric acid or sodium hydroxide.

In one embodiment, the active compound or compounds are prepared with carriers that will protect the therapeutic compounds against rapid elimination from the body, such as a controlled release preparation, including implants and microencapsulated delivery systems. Biodegradable, biocompatible polymers can be used, such as [poly(ethylene-co-vinyl acetate)], polyanhydrides, polyglycolide, collagen, poly(orthoesters), and polylactide [poly(lactic acid)]. Such preparations may be prepared using standard techniques or may be obtained commercially, e.g. from Alza Corporation and Nova Pharmaceuticals, Inc. They can be prepared according to methods known to those skilled in the art, according to the requirements and specifications as described in the European Pharmacopoeia.

The pharmaceutical compositions can be contained, for example, in bottles, blisters, ampoules, containers, packages, or dispensers, with, for example, instructions for administration.

The therapeutic dose is the amount of the compound sufficient to obtain beneficial or desired therapeutic results. This dose may be the same as or different from the prophylactic dose, which is the amount necessary to prevent occurrence of the symptoms of the disease or disease. The therapeutic dose can be administered in one or more administrations, applications, or doses. The therapeutic dose is specific to each compound, it may be administered one or more times a day, in addition, the therapy may be continuous or it may be applied one or more times a week. The selection of the therapeutic dose depends on the severity of the disease or disorder, the previous treatment administered, general health, and factors such as body weight, age of the patient and other accompanying diseases. Therefore, the therapeutic dose will be selected by one skilled in the art who determines the dosage and time required to effectively achieve therapeutic effects.

Dosage, toxicity, and therapeutic efficacy of compounds can be determined by standard procedures, which are *in vitro* cell culture experiments and *in vivo* animal experiments. For this purpose, the parameter LD50 (dose lethal to 50% of the population) and ED50 (dose therapeutically effective in 50% of the population) can be determined. The therapeutic index is determined and is expressed as the ratio LD50/ED50 from the ratio between doses having toxic and therapeutic effects. Compounds are considered therapeutically beneficial when their therapeutic indices are high. Another parameter used may be the IC50 (the concentration at which 50% of the tumor cell proliferation/viability is inhibited, relative to the control). In therapy, compounds exhibiting toxicity can be used, however, during the design stage, appropriate drug transport systems are then created that direct the molecules to the site of the disease. Such systems are designed to minimize the potential damage to normal cells and thus reduce side effects.

The data obtained from cell culture assays and animal studies can be used to construct a range of dosage in humans. Such dosage falls within the range of circulating concentrations of compounds for which little or no toxicity is observed. The dosage may vary within this range depending upon the dosage form employed and the route of administration utilized. For example, a therapeutically effective dose can be estimated initially from cell culture assays. The dose can then be tested in animal models where, for example, the IC50 value (i.e. the concentration of test compound that inhibited the symptoms by 50%) is determined. Additionally, the activity of the test compound can be compared with the activity of preparations used in human therapies against other diseases or pathological conditions. Such information can be used to determine therapeutic doses for humans.

### Description of the figures

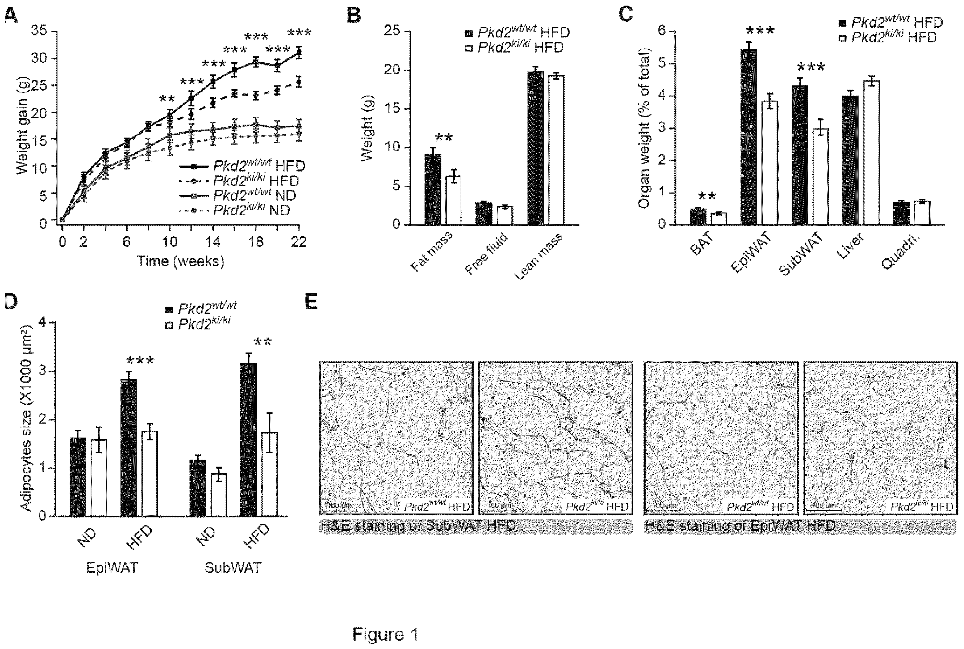

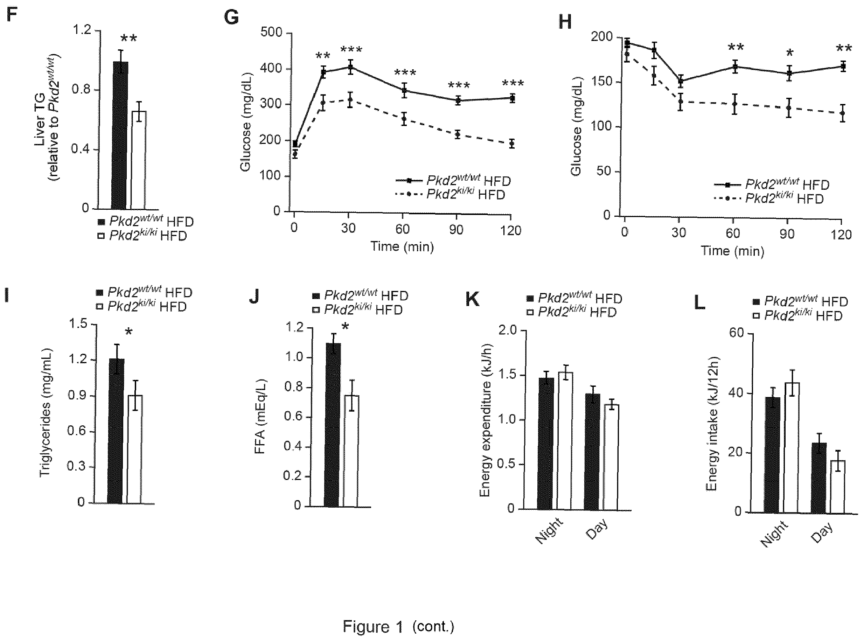

**Figure 1** **shows PKD2 inactivation effect. A.** Body weight gain of male mice with the specified genotypes on normal (ND) or high-fat diet (HFD). **B.** Quantification of fat, free fluid and lean mass by nuclear magnetic resonance (NMR) of mice on HFD in Fig 1A. **C.** Organ weight (percentage of total weight) of different fat depots, liver and quadriceps of *Pkd2*^{*wt*/*wt*} and *Pkd2*^{*ki*/*ki*} male mice after 22 weeks on HFD. **D.** Quantification of the average adipocyte size in SubWAT and EpiWAT of male mice of the specified genotypes on normal and high-fat diet. E. Representative images of H&E staining of SubWAT and EpiWAT of indicated HFD fed male mice. **F.** Triglyceride content in liver of *Pkd2*^{*wt*/*wt*} and *Pkd2*^{*ki*/*ki*} male mice in Fig 1A, relative to *Pkd2*^{*wt*/}*^{wt}.* **G.** Glucose tolerance test of the specified genotypes after 16 weeks on HFD. **H.** Insulin tolerance test of *Pkd2*^{*wt*/*wt*} and *Pkd2*^{*ki*/*ki*} male mice after 18 weeks on HFD. I. Triglycerides and **J.** free fatty acids in circulation of specified mice after 18 weeks on HFD. **K.** Energy expenditure and L. energy intake of mice after 20 weeks on HFD. Male mice were subjected to ND or HFD directly after weaning and the specified metabolic parameters were measured. n=7 for ND. On HFD, n=7 for *Pkd2*^{*wt*/*wt*} and n=8 for *Pkd2*^{*ki*/}*^{ki}.* Data presented as average +/- SEM,* P≤0.05, ** P ≤0.01, *** P ≤0.001.

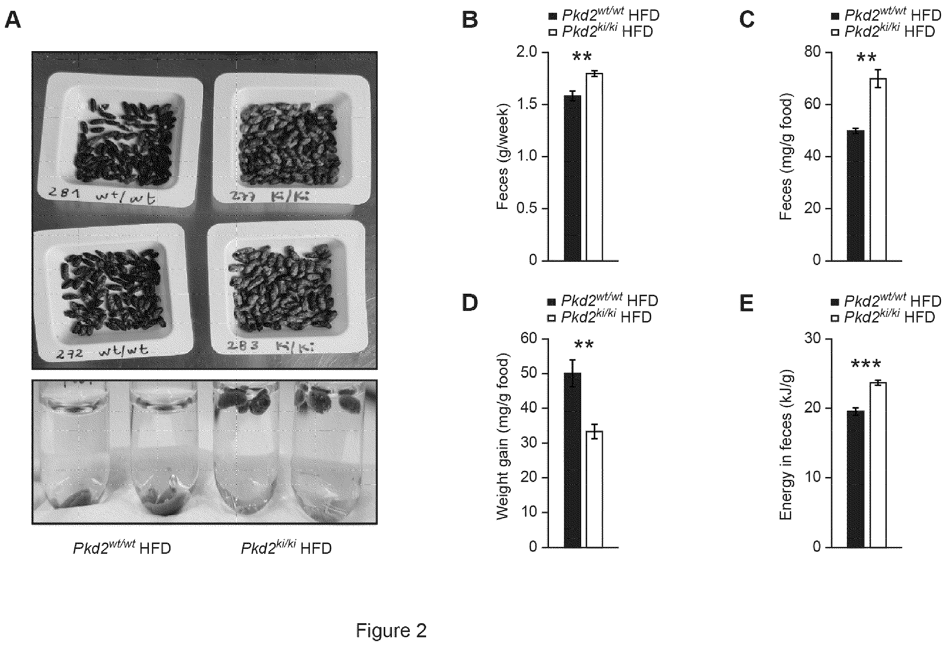

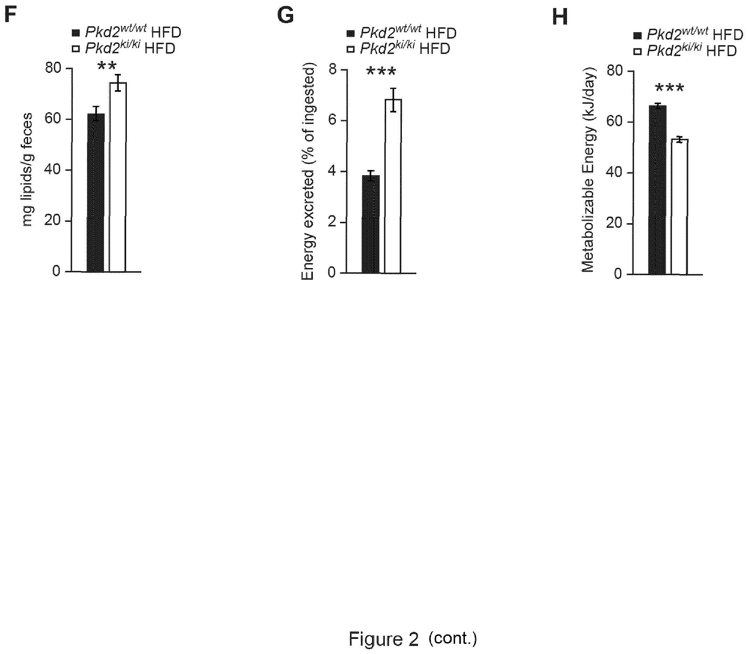

**Figure 2** **shows that *Pkd2* knock-in mice excrete more energy in feces and present lower absorption of lipids in the intestine. A.** Images of feces collected from male mice fed HFD and an image of them placed in water. **B.** Weight of feces collected in a week. **C.** Feces excreted per gram of food consumed. **D.** Body weight gained per gram of food consumed. E. Energy content and **F.** lipids content per gram of feces. **G.** Percentage of energy excreted in feces from the total energy ingested. **H.** Metabolizable energy calculated from intake minus excreted and assuming a urinary excretion of 2%. For Fig. 2A-H, male mice after weaning were kept in individual cages during two weeks and fed with HFD. For the first two weeks animals were acclimatized in the cages and then during two more weeks mice were monitored for food consumption and feces were analyzed for deposition of lipids and energy. n=7 for *Pkd2*^{*wt*/*wt*} and n=8 for *Pkd2*^{*ki*/}*^{ki}.* Data presented as average +/- SEM,* P≤0.05, ** P ≤0.01, *** P ≤0.001.

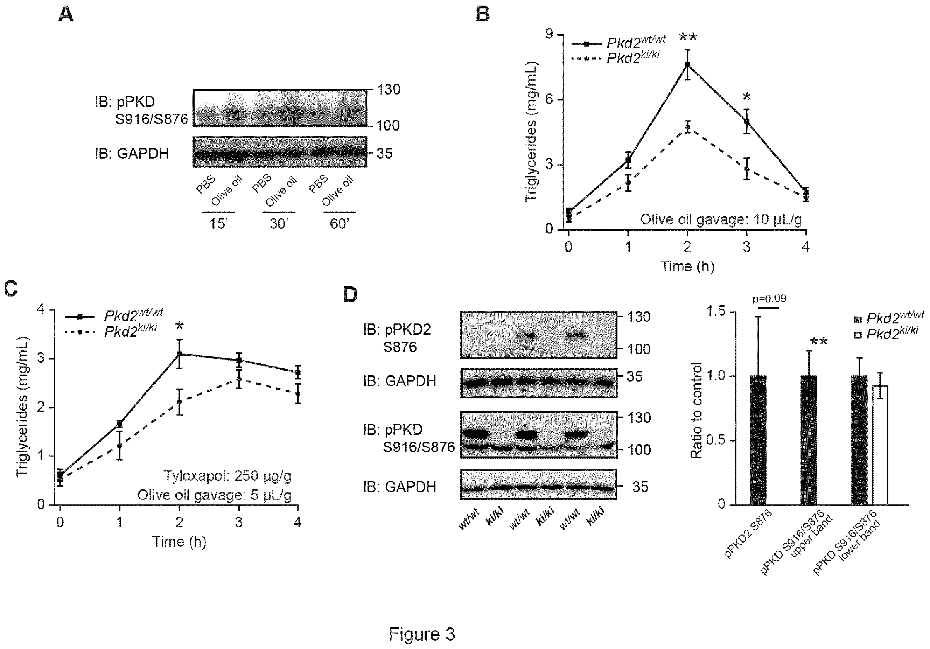

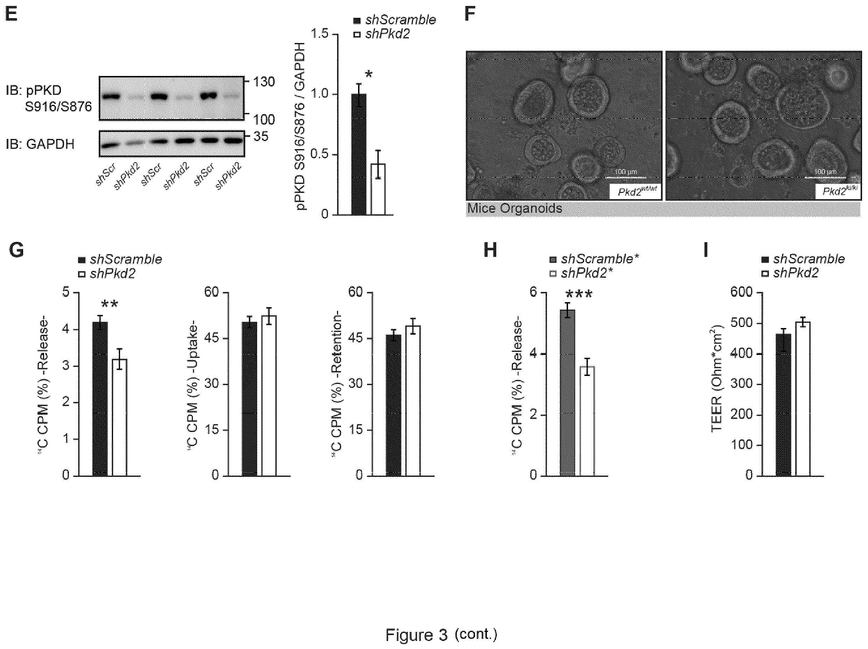

**Figure 3** **shows that PKD2 inactivation decreases lipids' absorption. A.** Western blot analysis of phosphorylated PKD (S916/S876) in small intestine of male C57BL/6 mice after overnight fasting, olive oil gavage (or PBS in controls) and dissection at different time-points. **B.** Lipids tolerance test after oral gavage of olive oil (10µL per gram of body weight). n=6. **C.** Lipids tolerance test 30 min after tyloxapol i.p. injection (250µg/g body weight). In this case, only 5µL of olive oil per gram of body weight were gavaged orally. n=6. **D.** Western blot analysis and quantification of phosphorylated PKD2 (S876) and phosphorylated PKD (S916/S876) in small intestine of *Pkd2*^{*w*/*lwt*} and *Pkd2*^{*ki*/*ki*} male mice. n=3. E. Western blot analysis and quantification of phosphorylated PKD (S916/S876) in Caco2 cells expressing *shscramble* or *shPkd2.* n=3. **F.** Representative light microscopy pictures of organoids derived from jejunum crypts of indicated male mice. **G.** Percentage of released, uptaken and retained ¹⁴C-labeled fatty acids from Caco2 cells *shscramble* or *shPkd2* (sequence 1) in a transwell system. n=6. **H.** Percentage of released ¹⁴C-labeled fatty acids by Caco2 cells expressing *shscramble** or *shPkd2** (sequence 2) grown in a transwell system. n=6. I. Transepithelial electric resistance of the Caco2 cells *shscramble* or *shPkd2* (sequence 1). n=6. For Fig 2B-C, after acclimation, male mice after were treated as specified and triglycerides in circulation measured at the specified time-points. Data presented as average +/- SEM,* P≤0.05, ** P ≤0.01, *** P ≤0.001.

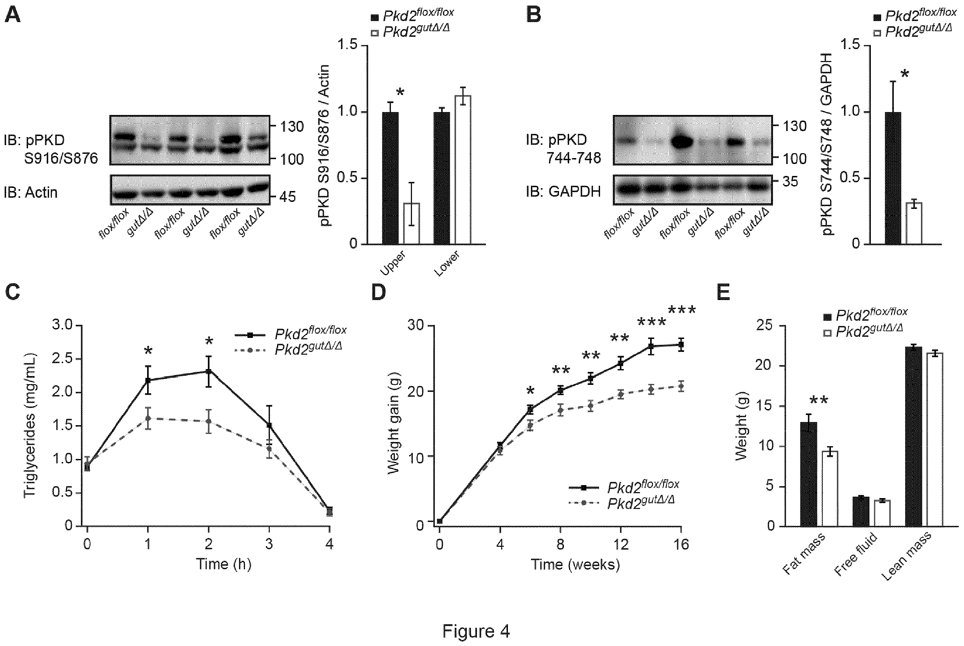

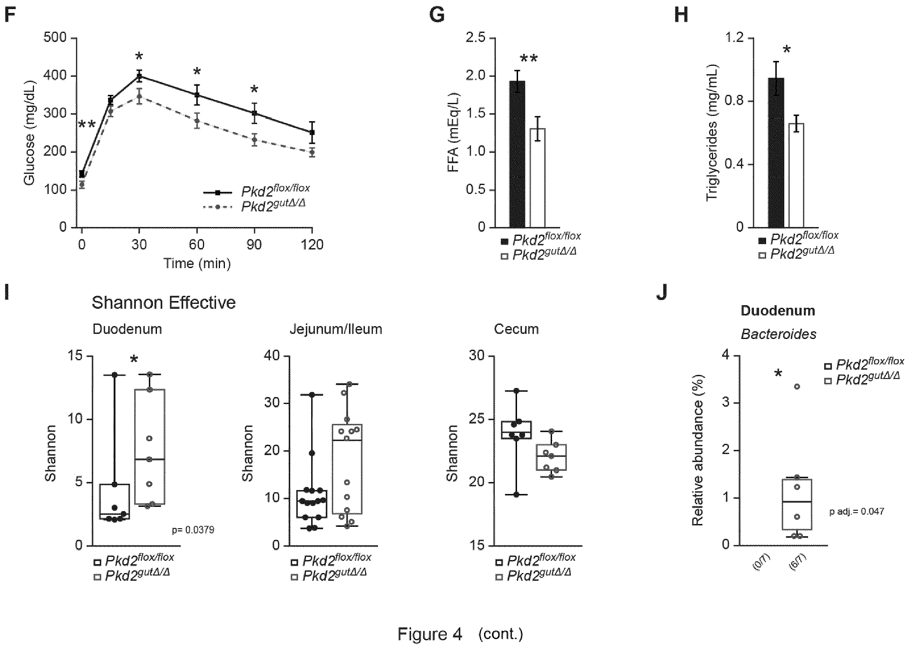

**Figure 4** **shows how intestinal deletion of PKD2 reduces lipids absorption, protects from HFD-induced obesity and improves microbiota profile. A.** Western blot and quantification of phosphorylated PKD (S916/S876) in small intestine of *Pkd2*^{*flox*/*flox*} and *Pkd2*^{*glutΔ*/*Δ*} male mice. n=3. **B.** Western blot and quantification of phosphorylated PKD (S744-748) in small intestine of *Pkd2*^{*flox*/*flox*} and *Pkd2*^{*gutΔ*/*Δ*} male mice. n=3. **C.** Lipids tolerance test after oral gavage of olive oil to *Pkd2*^{*flox*/*flox*} and *Pkd2*^{*gutΔ*/*Δ*} male mice after 6 weeks on HFD. n=10. **D.** Body weight gain of male mice of indicated genotypes on HFD. n=10. E. Quantification of fat, free fluid and lean mass by nuclear magnetic resonance of mice in Fig 4B. n=10. **F.** Glucose tolerance test of male mice with indicated genotypes after 16 weeks on HFD. n=10. **G.** Free-fatty acids and **H.** Triglycerides content in serum of male mice after 17 weeks on HFD. n=10. I. Differences in bacterial alpha diversity as indicated by Shannon Index. n=7. **J.** Changes in specific Operational taxonomic unit (OTU) in small intestinal samples. n=7. Data presented as average +/- SEM,* P≤0.05, ** P ≤0.01, *** P ≤0.001. In Fig. 3G Statistical testing was performed using Mann Whitney U test. In Fig 3H Statistical testing was performed using PERMANOVA with corrections for multiple testing using the Benjamini & Hochberg method.

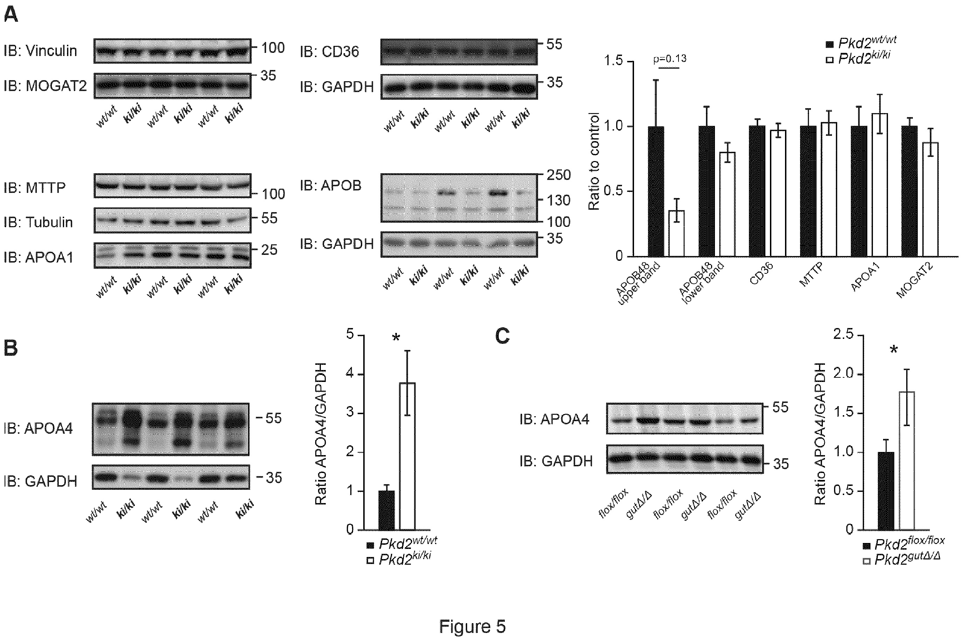

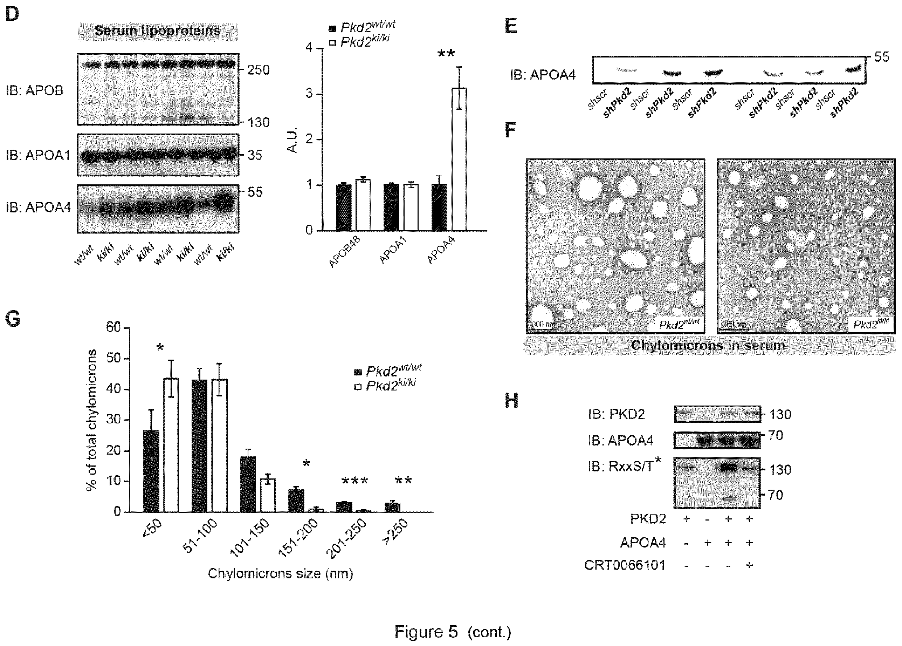

**Figure 5** **shows that PKD2 is associated with chylomicron size. A.** Western blot (WB) analysis of specified proteins in small intestine of *Pkd2*^{*wt*/*wt*} and *Pkd2*^{*ki*/*ki*} male mice after one week of HFD. Quantification of the bands for each protein normalized to loading control and relative to *Pkd2*^{*wt*/}*^{wt}.* n=3. **B.** WB of ApoliproteinA4 in jejunum of *Pkd2*^{*wt*/*wt*} and *Pkd2*^{*ki*/*ki*} male mice after one week of HFD. Quantification of bands normalized to loading control and relative to *Pkd2*^{*wt*/}*^{wt}.* n=3. **C.** WB of ApoliproteinA4 in jejunum of *Pkd2*^{*flox*/*flox*} and *Pkd2*^{*gutΔ*/*Δ*} male mice after one week of HFD. Quantification of bands normalized to loading control and relative to *Pkd2*^{*flox*/*flox*} n=7. **D.** WB analysis of specified apolipoproteins in 10µL of serum of overnight fasted, 2 hours refed male animals after 1 week on HFD. Quantification of bands relative to *Pkd2*^{*wt*/}*^{wt}.* n=4. E. WB of 20µL of basal medium of Caco2 cells expressing *shscramble* or *shPkd2* in transwell system after 12 hours stimulation with oleic acid. n=6. **F.** Electron microscopy pictures of chylomicrons obtained by ultracentrifugation of plasma from *Pkd2*^{*wt*/*wt*} and *Pkd2*^{*ki*/*ki*} male mice as specified in methods. **G.** Quantification of the diameter the chylomicrons shown in Fig 5F. n=3. **H.** WB with specified antibodies after an in-vitro kinase assay. Data presented as average +/- SEM,* P≤0.05, ** P ≤0.01, *** P ≤0.001.

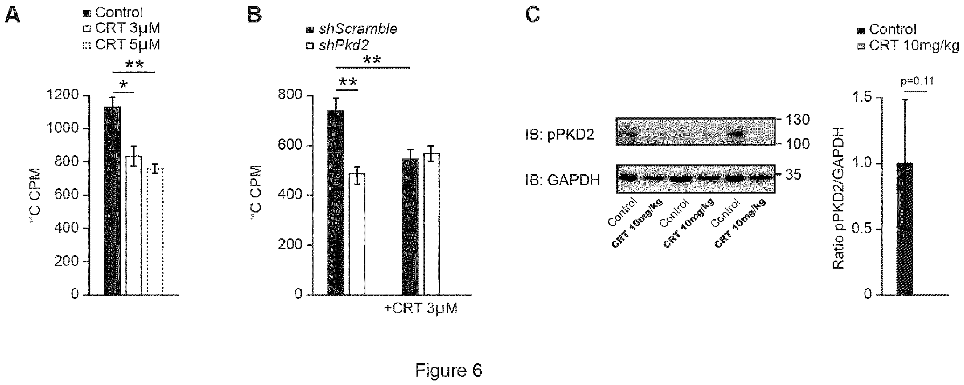

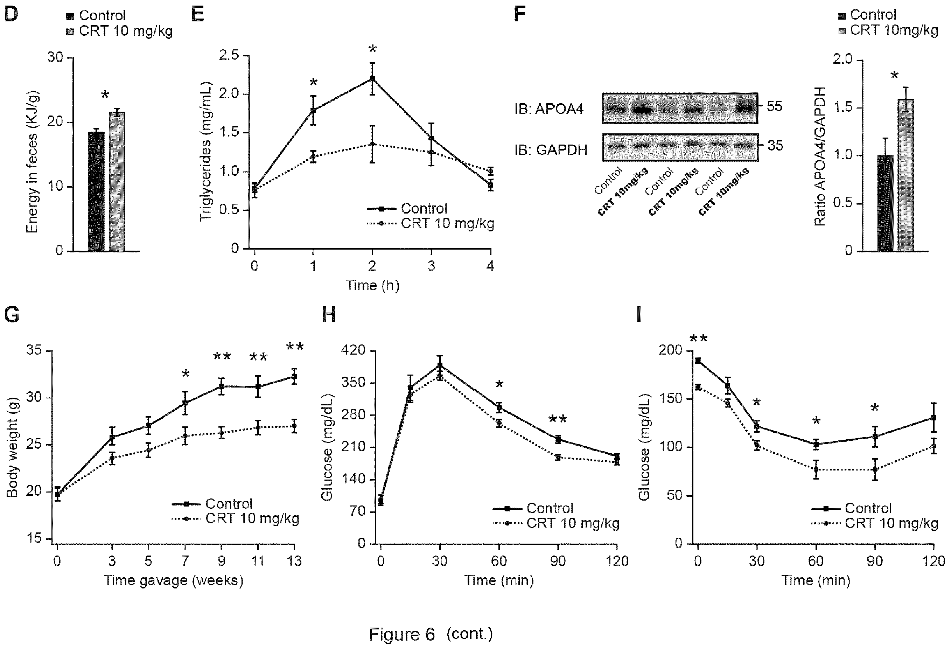

**Figure 6** **shows that inhibition of PKDs by small-molecule compound reduces the degree of diet-induced obesity and diabetes. A.** Basolateral release of ¹⁴C-labeled fatty acids in Caco2 cells grown in a transwell system and treated with CRT0066101 at the indicated concentrations for 24 hours. n=6. **B.** Basolateral release of ¹⁴C-labeled fatty acids in Caco2 cells expressing *shscramble* or *shPkd2* in a transwell system and treated with CRT0066101 for 24 hours. n=8. **C.** Western blot analysis of phosphorylated PKD2 in jejunum of male C57BL/6 mice on HFD and treated with CRT0066101 or control (water) for 13 weeks. n=3. **D.** Energy content per gram of feces of male mice in HFD and receiving daily gavage of CRT0066101 or water as control. n=6 for control and n=5 for CRT0066101. **E.** Lipids tolerance test after oral gavage of olive oil to male mice after 9 weeks in HFD and receiving daily CRT0066101 or control. n=6 for control and n=5 for CRT0066101. **F.** WB of Apolipoprotein A4 (APOA4) in jejunum of male C57BL/6 mice on HFD and treated with CRT0066101 or control (water) for 13 weeks. Quantification of bands normalized to GAPDH and relative to control (water) n=6. **G.** Body weight of male C57BL/6 mice on HFD and treated with CRT0066101 or control (water) for 13 weeks. n=6 for control and n=5 for CRT0066101. **H.** Glucose tolerance test in male C57BL/6 mice which received CRT0066101 or water (control). Measurements after 10 weeks of treatment and HFD feeding. n=6 for control and n=5 for CRT0066101. I. Insulin tolerance test in male C57BL/6 mice which received CRT0066101 or water (control). Measurements after 11 weeks of treatment and HFD. n=6 for control and n=5 for CRT0066101. Data presented as average +/- SEM,* P≤0.05, ** P ≤0.01.

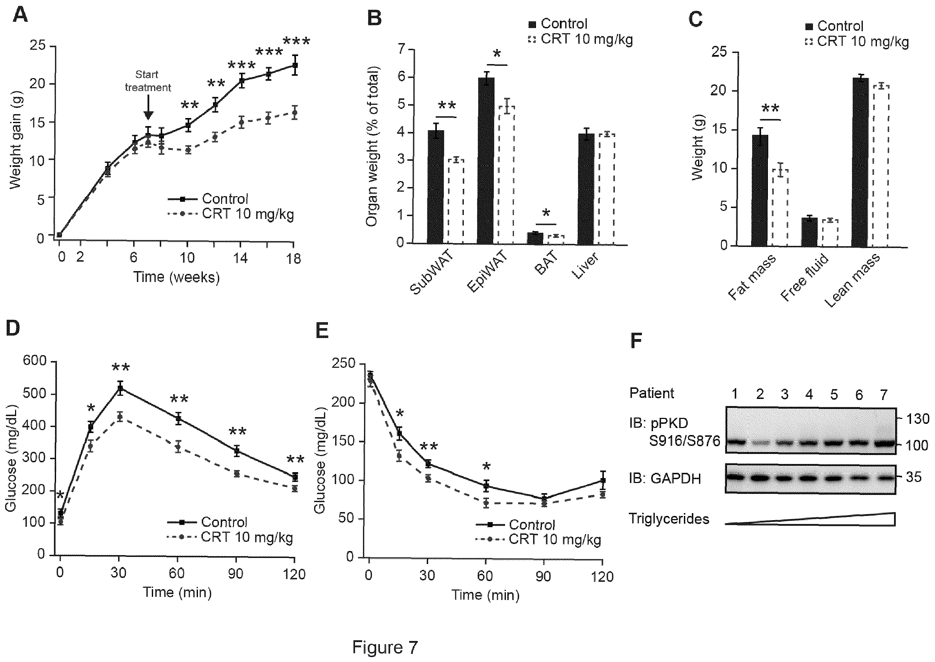

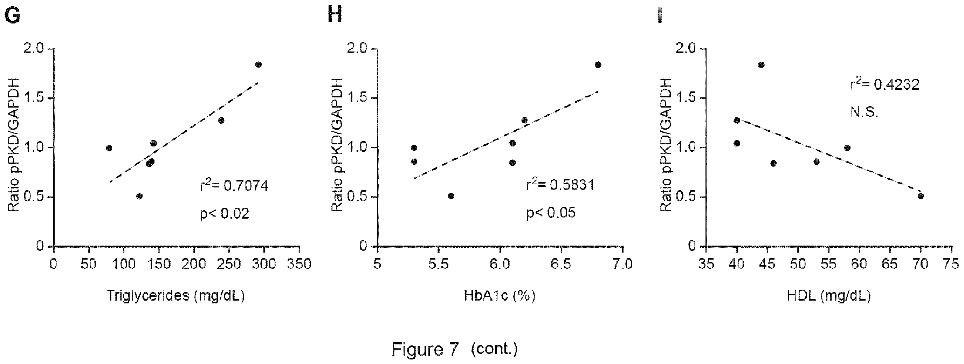

**Figure 7** **shows that reduction of PKD activity in intestine ameliorates pre-established obesity in mice and correlates with healthier blood profile in obese patients. A.** Body weight gain of male C57BL/6 mice that, after 7 weeks on HFD, started receiving treatment with CRT0066101 or water. HFD continued along with treatment. n=8. **B.** Organ weight (expressed as percentage of total body weight) of different fat depots and liver of male mice in Fig 7A. **C.** Quantification of fat, free fluid and lean mass by nuclear magnetic resonance (NMR) of male mice in Fig 7A. **D.** Glucose tolerance test 8 weeks after starting the treatment of mice in Fig 7A. E. Insulin tolerance test 10 weeks after starting the treatment of mice in Fig 7A. **F.** Western blot analysis of PKD phosphorylation in proximal jejunum of morbidly obese female patients. Samples are loaded according to fasting triglyceride levels (lower to higher). n=7. **G.** Scatter plot of PKD phosphorylation in jejunum (normalized to GAPDH) vs. triglycerides of morbidly obese female patients. n=7. **H.** Scatter plot of PKD phosphorylation in jejunum (normalized to GAPDH) vs. percentage of glycated hemoglobin (HbA1c) of morbidly obese female patients. n=7. **I**. Scatter plot of PKD phosphorylation in jejunum (normalized to GAPDH) vs. high density lipoprotein (HDL) of morbidly obese female patients. n=7. Data presented as average +/-SEM,* P≤0.05, ** P ≤0.01, *** P ≤0.001. In Fig 7G-I, r²= square of the Pearson product-moment correlation coefficient. p determined for Pearson correlation coefficient for the given degrees of freedom.

### EXAMPLES

### Methods

### Generation of mouse models

All animal experiments were approved by the local institutional animal care (Regierung von Unterfranken, Germany) and conducted according to the guidelines and state regulations. Experiments were performed under animal protocol numbers AK 55.2-2531.01-124/13 and 55.2-2532-2-741. Mice were maintained in a specific-pathogen-free facility with the ambient temperature set at 23°C, following a 12-h light-dark cycle and given ad libitum access to water and standard chow diet which was exchanged under indicated experimental conditions to High-fat diet (HFD). All mice were closely monitored by the inventors, facility technicians and by an independent veterinary scientist responsible for animal welfare. Euthanasia was performed by cervical dislocation, in a separate area, away from other animals and all efforts were made to minimize suffering. No animals died or became ill during the development of this research.

*Pkd2* knockin mice were obtained from The Jackson Laboratories (Matthews, Navarro et al., 2010b). These mice were maintained in a C57BL/6 background and presented point mutations in Ser⁷⁰⁷ and Ser⁷¹¹ which were mutated to Alanines (*Pkd2*^{*ki*/*ki*}). The generation of mice bearing a specific deletion of intestinal PKD2 was achieved by crossbreeding Villin-Cre mice (B6.Cg-Tg(Vil1-cre)1000Gum/J) with *Pkd2*^{flox/flox} mice (Ishikawa, Kosako et al., 2016a). Genotyping was performed for indicated mice models following standard PCR protocols and using respected primer sets.

### Animal experiments

Mice body weight development was closely monitored and reported here on a weekly basis when mice were on a specified diet. All experiments were performed in male mice.

### Oral administration of PKD inhibitor

PKD inhibitor CRT0066101 was dissolved in water (10mg/kg) and administered orally to the mice by daily gavage, while the control mice were gavaged with water. Mice were maintained on the specified diets and had *ab libitum* access to food and water. C57BL/6 mice were administered CRT inhibitor treatment at 4-weeks of age, while for the rescue experiment, inhibitor was given under the same circumstances but treatment started only after 7 weeks of HFD feeding. Intestinal barrier permeability was further assessed 1 hour after oral gavage of FITC-Dextran 4.

### Indirect calorimetry

Energy expenditure, food intake, and activity measurements were obtained in a PhenoMaster (TSE Systems) as previously described (El-Merahbi, Viera et al., 2020, Trujillo Viera, El-Merahbi et al., 2016). Briefly, mice were kept in separate metabolic cages with unlimited access to water and to the specified diet. After 48 hours of acclimation, data was collected every 10 min including photobeam breaks, oxygen consumption, carbon dioxide dissipation, and food and water consumption. Results in bars represent the average of each night/day cycle.

### Metabolic tests

A glucose tolerance test (GTT) was performed after overnight fasting. Mice blood glucose was assessed before and 15, 30, 60, 90, and 120 minutes after intraperitoneal injection of glucose (2 g/kg). For the Insulin tolerance test (ITT), the same measurements were performed after 4 hours of fasting and intraperitoneal injection of 0.5 U/kg of recombinant insulin. For glucose measurements, one blood drop was drawn from the tail tip into a test strip and measured with a glucometer (Accu-Chek^{®} Roche).

Glucose-stimulated insulin secretion was measured with the Ultra Sensitive Mouse Insulin ELISA Kit (Crystal Chem) according to the manufacturer's instructions. Briefly, overnight fasted mice were i.p. injected with 30 % glucose (3 g/kg), and blood samples were taken from the tail tip at 0, 2, 5, 15, and 30 min. 5 µL of serum were added to the antibody-coated microplate and incubated. Then, the second reaction with the anti-insulin enzyme conjugate and the third reaction with the enzyme-substrate were performed before measuring absorbance at 450 nm and 630 nm. Calculations were performed according to the Low Range Assay (0.1-6.4 ng/mL) standards.

Lipids tolerance test was performed as described previously (Wang, Rong et al., 2016b). Briefly, overnight fasted mice were gavaged with olive oil (10 µL/g body weight). Blood was collected at indicated times for measurement of serum triglycerides content. For lipids absorption under tyloxapol treatment, 250 µg/g of body weight were i.p. injected to mice 30 minutes before the gavage of olive oil 5 µL/g body weight.

Triglycerides, free-fatty acids, and glycerol in circulation were determined in serum samples using the mentioned kits and according to their manufacturers' instructions.

Liver triglycerides were measured by homogenization of 50 mg of liver in 500 uL of lysis buffer and lipids extraction in methanol:chloroform, phase extraction and evaporation. The lipids were redissolved in DMSO and triglycerides quantified with the mentioned kit and according to the manufacturers' instructions.

### Feces analysis

The bedding of single-caged mice was collected and the ingested food ingestion from the individual mice was assessed. All the stools were manually collected. After overnight dissecation at 60°C, the samples were weighted and frozen for further analysis. The amount of feces produced per mouse was compared to the amount of food consumed and the weight gain. About 3 g of dried feces material were homogenized and 1 g was pressed into a tablet and accurately weighted. Calorie content from feces and diet was measured in duplicates in a 6400 Automatic Isoperibol Calorimeter (Parr Instrument Company). To analyze energy excreted, the amount of energy in feces was compared to the amount of energy ingested (the product between grams of food and caloric content of the food). Lipids extraction from feces was performed as previously described (Kraus, Yang et al., 2015).

### DNA extraction and 16S rRNA Sequencing

We collected cecal contents as well as mucosal scrapings from the small intestine, immediately snap froze in liquid nitrogen, and stored them at -80 °C. DNA Isolation, library preparation, and sequencing were performed by the ZIEL - Core Facility Microbiome of the Technical University of Munich. Briefly, DNA was extracted using previously published protocols (Klindworth, Pruesse et al., 2013). For the assessment of bacterial communities primers specifically targeting the V3-V4 region of the bacterial 16S rRNA gene were used including a forward and reverse illumina specific overhang and a barcode. Sequencing was performed using an Illumina MISeq DNA platform. Multiplexed sequencing files were analyzed using the IMNGS platform, based on the UPARSE approach for sequence quality check, chimera filtering, and cluster formation (Edgar, 2013). For the analyses, standard values for barcode mismatches, trimming, expected errors, and abundance cutoff were used and only sequences between 300 and 600 bp were included for analyses. Downstream analyses of the IMNGS platform output files were performed using the RHEA R pipeline (Lagkouvardos, Fischer et al., 2017). In brief, we normalized the abundances and assessed the quality of sequences using rarefaction curves (McMurdie & Holmes, 2014). The analysis of alpha and diversity beta diversity as well as group comparison were performed using default settings with an exception for the group comparisons, where we excluded alpha diversity from the analysis and set the prevalence cutoff value to 0.5. For presentation, the obtained graphical output was modified using inkscape (https://inkscape.org). Assignment of OTUs to taxons was performed using the SILVA database(Quast, Pruesse et al., 2013).

### Cell culture and stable cell lines

Caco2 cells were cultured in Dulbecco's modified eagle's medium 4,5 g/L glucose (DMEM), 10% fetal bovine serum (FBS), non-essential amino acids (NEAA), 1 mM sodium pyruvate, and 40 µg/mL gentamycin. For the generation of shRNA cell lines, *Pkd2* shRNA lentiviral sequences or scramble (non-targeting) were first cloned into the pLKO.1-puro vector then packed into viral particles in packaging cells (HEK293T) using 3^{rd} generation packaging vectors. Caco2 cells were spinfected with viral supernatant then selected with puromycin and the deletion efficiency was further assessed by western blot.

### Lipid uptake in-vitro

Lipid transport studies were assessed in Caco2 cells seeded in trans-wells. 1.5x10⁵ cells were seeded into a 12-well cell culture insert of 1µm pore size Trans-well and maintained in for 14 consecutive days with media change every two days. For the lipids uptake and release, the apical side of the membrane was loaded with radioactive ¹⁴C-palmitic acid (0.2 µCi), oleic acid (1 mM), and taurocholic acid (2 mM) in DMEM high glucose 10% FBS. The basal medium contained DMEM high glucose 0.1 % FBS. After 24 hours of incubation, cell lysates and the apical and basolateral medium was collected and the ¹⁴C-palmitic acid was measured in a scintillation counter for aliquots of apical and basal medium and for cell lysate. The same protocol was used in Caco2 cells that were treated with CRT0066101 (3 and 5 µM) for 24 h (added together with the mentioned radioactive fatty acid cocktail).

### Transepithelial electrical resistance

Barrier integrity of the established Caco-2 *in vitro* models was determined by chop stick hand-electrode (Millicell ERS-2, Millipore, Billerica, MA) as previously described by Srinivasan et al. (Srinivasan, Kolli et al.). TEER (transepithelial electrical resistance) was indicated as Ω*cm² for models setup with *shScramble* and *shPkd2* Caco-2 cells after 14 days of differentiation to enterocyte-like Caco-2 cells. The permeability of Caco-2 cells monolayer was further assessed by using fluorescein isothiocyanate dextran 4,400 (FD-4) as the model compound for paracellular tight junction transport.

### In-vitro kinase assay

Recombinant human proteins used for the study, PKD2 and APOA4, were both purchased from Abcam. Kinase reactions were carried out in a kinase reaction buffer containing the immune complex, recombinant proteins, and ATP as described in (Bedford, Kasper et al., 2011). Then, western blot was performed and incubated with a primary antibody against the phosphorylated motif (RxxS/T*) (Cell Signaling).

### Western Blot

Proteins from tissues and cell culture were extracted with RIPA buffer supplemented with phosphatase and protease inhibitor (PPI, Thermo Fisher Scientific). Protein concentration was measured by Pierce^{™} BCA Protein Assay Kit (Thermo Fisher Scientific) following the manufacturer's protocol. Reduced protein extracts were separated on 10 % SDS-PAGE gels by electrophoresis and transferred to PVDF membranes under wet transfer. Membranes were blocked in 5 % (w/v) milk in TBS-T before overnight probing with indicated primary antibodies at 4°C, followed by TBS-T washes and incubation with corresponding secondary antibody. The signals were detected with an enhanced chemiluminescence solution in an Amersham Imager 680 (GE Healthcare) and automatically quantified by the software according to the manufacturer's instructions.

### Chylomicrons analysis

A study of chylomicrons size was performed as previously reported (Wang et al., 2016b) with some modifications. Mice on HFD were overnight fasted and given a bolus of olive oil (10 µL/g of body weight) 2 hours before sampling blood. Plasma from three mice was pooled and 200 µL were diluted with saline 1:4 ratio. The mixture was centrifuged for 3 h at 50,000 rpm and 4°C. The top layer containing the chylomicrons was then removed and 5 µL were applied to the carbon-coated copper grids. Chylomicrons were stained with uranyl acetate for 15 min and visualized under electron microscopes Zeiss EM 900 and 80 kV (50,000x). Quantification of the chylomicrons size was done with ImageJ from 4 different pictures per genotype.

### Mice organoids culture

For a generation of mice organoids, 1-2 cm of jejunum from *Pkd2*^{*wt*/*wt*} and *Pkd2*^{*ki*/*ki*} mice were washed with HBSS and cut open. Mucus and villi were removed with HBSS and by scraping with glass slides. Then, a series of 6 washings with cold HBSS and shaking (in order: vortex 5 sec, rotation 30 min, inversion, and 3x manual shaking) ensure the removal of the villi and extraction of the crypts. After centrifugation, crypts are seeded in Matrigel^{™} (5000 crypts/mL) in 50 µL drops. After an incubation period of 10-20 min at 37°C, 300µL culture medium was added to cover the solidified drops. Basal medium, for resuspension of pellets, contains DMEM-F12 Advanced complemented with 1x N2, 1x B27 w/o vitamin A, 1x Anti-anti, 10mM HEPES, 2mM GlutaMAX-I, 1mM N-acetylcystein. The organoids were maintained in basal medium supplemented with 500ng/mL hR-Spondin 1, 100ng/mL rec Noggin, 50ng/mL hEGF, 3µM CHIR99021 and 1mM valproic acid (also 10µM Y-27632 only the first day after splitting). Medium was changed every 2 days.

### Histological analysis

For organoids imaging studies, mice organoids were collected and, after removal of the Matrigel^{®} matrix (Corning), they were fixed with 4% paraformaldehyde for 1h at 4°C and resuspended in Histogel^{™} Drops were embedded in paraffin, sectioned, and deparaffinized. Antigen retrieval was performed with a steamer and blocking with 5% normal serum. Organoids are then incubated with primary antibodies against indicated antigens followed by fluorescent labeling with appropriate secondary antibodies. Samples were mounted using Fluoromount-G^{™} with DAPI. Slides were visualized using Leica TCS SP8 confocal microscope (Sato, Vries et al., 2009).

Adipose tissues, liver, and intestine were dissected and directly placed to paraformaldehyde 4% at 4°C for 24h fixation. Tissues were dehydrated. Samples were embedded in paraffin and cut into 5 µm sections. Standard Hematoxylin-Eosin staining was performed and digital pictures were taken in a Leica light microscope DM4000B at 20x for adipose tissue, 40x for liver sections, and 10x for intestine. A blinded experiment was performed to measure adipocyte size. 6 specimens per genotype and about 400-500 adipocytes per specimen were measured with ImageJ for that purpose.

For pancreatic islets stainings and quantification, the pancreas was dissected and directly embedded into OCT. 3 different sections (7 µm) per subject were taken with a distance of 50 µm. Sections were fixed (4% PFA) and blocked (5% BSA, 0.2% Triton in PBS) before overnight incubation (4°C) with the primary antibody. Then, the sections were washed and incubated with the secondary antibody (1 hour at RT) before mounting with DAPI.

### Real-time PCR analysis

Total RNA was extracted from tissues and cells using QIAzol Lysis Reagent (QIAGEN) according to the manufacturer's instructions. 1 µg of RNA was reversely transcribed with the First Strand cDNA Synthesis Kit (Thermo Fisher Scientific) according to their protocol. cDNA was then diluted 1:15 and used for qPCR with SYBR Green. The absolute quantification of PKD isoform copy numbers was performed according to a standard protocol from Applied Biosystems. In brief, primers were designed to be located within same exons and genomic DNA of known concentration was used for creating a standard curve reflecting copy numbers.

### Human samples

Fasting blood samples were collected from 7 morbidly obese (BMI 42.7-54 kg/m²) female patients aged 33-57 years prior to Roux-en-Y gastric bypass (RYGB) surgery for standard biochemical analysis. A whole-wall segment of proximal jejunum (5cm) was collected from fasted patients during the surgery and gently rinsed in ice-cold PBS before rapidly snap-freezing in liquid nitrogen. At the time of surgeries, two patients were on metformin medication for type 2 diabetes and one patient for polycystic ovary syndrome. Prior to surgeries, all patients observed a standard 2-4 week very low calorie diet (1, 000 kcal per day) which comprised 2 daily liquid meals (such as egg white shakes or vegetable soup) and 1 daily solid meal (such as tuna fish or chicken breast salads) that were low in fat and carbohydrates and rich in protein (100g). Informed, written consent was obtained from all patients and usage of human material was approved by the Ethics Committee of the Medical Faculty of the University of Würzburg (approval number EK AZ 188/17 - MK).

### Statistical Analyses

The results are presented as mean values ± standard error of the mean (SEM). Significances were assessed by using a two-tailed Student's t-test for independent groups. P values of 0.05 or lower were considered as statistically significant (*p<0.05, **p<0.01, and ***p<0.001).

For microbiota studies, analysis of alpha diversity was performed with GraphPad prism v6 using the non-parametric Mann-Whitney U test. We visualized beta diversity using non-metric multidimensional scaling based on generalized UniFrac and tested for significance using PERMANOVA. Differences in OTUs were determined using Fisher's Exact test with adjustments for multiple testing using the Benjamini & Hochberg method.

For scatter plots of human samples, r²= square of the Pearson product-moment correlation coefficient and p was determined for Pearson correlation coefficient for the given degrees of freedom.

**Table 1. List of primers**

| **Gene** | **Forward (5'-3')** | **Reverse (5'-3')** |
|---|---|---|
| *Pnlip* | CTGGGAGCAGTAGCTGGAAG | AGCGGGTGTTGATCTGTGC |
| *Clps* | GAACAGTATGCAGTGTAAGAGCA | GCAGATGCCATAGTTGGTGTTG |
| *Cyp7a1* | ATCAAAGAGCGCTGTCTGGGT | GCGTTAGATATCCGGCTTCAAAC |
| *Cyp8b1* | CTAGGGCCTAAAGGTTCGAGT | GTAGCCGAATAAGCTCAGGAAG |
| *Abcg5* | AGGGCCTCACATCAACAGAG | GCTGACGCTGTAGGACACAT |
| *Abcg8* | CTGTGGAATGGGACTGTACTTC | GTTGGACTGACCACTGTAGGT |
| *Ehhadh* | ATGGCTGAGTATCTGAGGCTG | GGTCCAAACTAGCTTTCTGGAG |
| *Acaa1a* | TCTCCAGGACGTGAGGCTAAA | CGCTCAGAAATTGGGCGATG |
| *Cd36* | ATGGGCTGTGATCGGAACT | GTCTTCCCAATAAGCATGTCTCC |
| *Bacs* | TCTATGGCCTAAAGTTCAGGCG | CTTGCCGCTCTAAAGCATCC |
| *Baat* | GGAAACCTGTTAGTTCTCAGGC | GTGGACCCCCATATAGTCTCC |
| *Taat* | GCACACGGCCTGAAGATGA | ATIIIIGTAGCAGAGGTACGGG |
| *Csad* | CCAGGACGTGTTTGGGATTGT | ACCAGTCTTGACACTGTAGTGA |
| *Asbt* | GTCTGTCCCCCAAATGCAACT | CACCCCATAGAAAACATCACCA |
| *Osta* | AGGCAGGACTCATATCAAACTTG | TGAGGGCTATGTCCACTGGG |
| *Ostb* | AGATGCGGCTCCTTGGAATTA | TGGCTGCTTCTTTCGATTTCTG |
| *Pppara* | AACATCGAGTGTCGAATATGTGG | CCGAATAGTTCGCCGAAAGAA |
| *Acadl* | TCTTTTCCTCGGAGCATGACA | GACCTCTCTACTCACTTCTCCAG |
| *Fabp4* | GGATGG444GTCGACCACAA | TGGAAGTCACGCCTTTCATA |
| *Fabp2* | GTGGAAAGTAGACCGGAACGA | CCATCCTGTGTGATTGTCAGTT |
| *Fatp4* | ACTGTTCTCCAAGCTAGTGCT | GATGAAGACCCGGATGAAACG |
| *Fatp2* | TCGTGGGACTGGTAGATTTTG | CGCGATGTGTTGAAAGAGTTT |
| *Pkd1* | GGGGGCATCTCGTTCCAT | GTGCCGAAAAAGCAGGATCTT |
| *Pkd2* | GCTGAGACACCTGCACTTCA | GGATCGGCTGATGCCAGTAA |
| *Pkd3* | GTCTGTCAAATGTATCTCTGCCA | GGTGAGTATGTGACTCTTCACTG |
| *Mttp1* | CTCTTGGCAGTGCIIIITCTCT | GAGCTTGTATAGCCGCTCATT |
| *Mogat2* | TGGGAGCGCAGGTTACAGA | CAGGTGGCATACAGGACGGA |
| *Dgat1* | GCGCTACTTCCGAGACTACTT | GGGCCTTATGCCAGGAAACT |
| *Apoa4* | GCATCTAGCCCAGGAAACTG | ATGTATGGGGTCAGCTGGAG |
| *Apoa1* | GGCACGTATGGCAGCAAGAT | CCAAGGAGGAGGATTCAAACTG |
| *ApoB* | AAGCACCTCCGAAAGTACGTG | CTCCAGCTCTACCTTACAGTTGA |
| *Ucp1* | AGGCTTCCAGTACCATTAGGT | CTGAGTGAGGCAAAGCTGATTT |
| *Cidea* | TGACATTCATGGGATTGCAGAC | GGCCAGTTGTGATGACTAAGAC |
| *Bmp7* | ACGGACAGGGCTTCTCCTAC | ATGGTGGTATCGAGGGTGGAA |
| *Adrb3* | AGAAACGGCTCTCTGGCTTTG | TGGTTATGGTCTGTAGTCTCGG |
| *Prdm16* | CCACCAGCGAGGACTTCAC | GGAGGACTCTCGTAGCTCGAA |
| *Cidec* | ATGGACTACGCCATGAAGTCT | CGGTGCTAACACGACAGGG |
| *Pgc1a* | AGCGCCGTGTGATTTACGTT | CCGCAGATTTACGGTGCATT |
| *Myh2* | AAAGCTCCAAGGACCCTCTT | AGCTCATGACTGCTGAACTCAC |
| *Ckm* | CAGCACAGACAGACACTCAGG | GAACTTGTTGTGGGTGTTGC |
| *Mck* | GCAAGCACCCCAAGTTTGA | ACCTGTGCCGCGCTTCT |
| *Slc6a8* | TGCATATCTCCAAGGTGGCAG | CTACAAACTGGCTGTCCAGA |
| *Slc27a2* | TCCTCCAAGATGTGCGGTACT | TAGGTGAGCGTCTCGTCTCG |
| *Ucp3* | CTGCACCGCCAGATGAGTTT | ATCATGGCTTGAAATCGGACC |
| *Myh1* | TCTGCAGACGGAGTCAGGT | TTGAGTGAATGCCTGTTTGC |

| **Genotyping** | **Forward (5'-3')** | **Reverse (5'-3')** |
|---|---|---|
| Pkd2^{ki/ki} | AGTGGCACGTTCCCCTTCAATG | CTTTGCCCAATCCCTTACAGCCT |
| Vil-Cre/WT | GCCTTCTCCTCTAGGCTCGT | TATAGGGCAGAGCTGGAGGA |
| Vil-Cre/Tg | GCCTTCTCCTCTAGGCTCGT | AGGCAAATTTTGGTGTACGG |
| Pkd2-Flox | TGAAGGAAGTGTCTTGGGAGTCC CTGCTGTTTTAATAGC | TGTCTAGGAGGGGACATAACGAA CCTGAGGAAACGGATCGGC |

| **Absolute quantification** | **Forward (5'-3')** | **Reverse (5'-3')** |
|---|---|---|
| *Pkd1* | TTTAACTCCCGTTGGAGCGA | CACTGTTGTTTGGTGGGGAAC |
| *Pkd2* | ATGTCACCTACTTTGTGGGCG | TGGGCGCATCTTGGAGGATA |
| *Pkd3* | AGGCAGTAACCCACACTGTTT | TCTGCGCCACATCTAGTCCC |

**Table 2. Materials**

| **REAGENT or RESOURCE** | **SOURCE** | **IDENTIFIER** |
|---|---|---|
| **Antibodies** | | |
| Rabbit anti-GAPDH | Sigma-Aldrich | Cat# G9545, RRID:AB_796208 |
| Rabbit Anti-APOA4/Apo-AIV | Abcam | Cat# AB_231660 |
| Mouse anti-apolipoprotein A4 | Cell Signaling Technology | Cat# 5700, RRID:AB_10859038 |
| Rabbit anti-apolipoproteinB | Proteintech | Cat# 20578-1-AP, RRID:AB_10732938 |
| Rabbit anti-apolipoprotein A1 | Abcam | Cat# ab20453, RRID:AB_445592 |
| Rabbit Anti-MOGAT2 | Abcam | Cat# ab63156, RRID:AB_956147 |
| Rabbit Anti-DGAT1 | Abcam | Cat# ab54037, RRID:AB_869453 |
| Rabbit Anti-MTTP | Abcam | Cat# ab63467, RRID:AB_10672035 |
| Rabbit Anti-PKD3/PKC,clone (D57E6) | Cell Signaling Technology | Cat# 5655, RRID:AB_10695917 |
| Rabbit Anti-Protein Kinase D2, clone (EP1495Y) | Abcam | Cat# ab51250, RRID:AB_882058 |
| Rabbit Anti-Protein Kinase D2, clone (D1A7) | Cell Signaling Technology | Cat# 8188, RRID:AB_10829368 |
| Rabbit Anti-PKD/PKCµ | Cell Signaling Technology | Cat# 2052 RRID:AB_2800149 |
| Rabbit Anti-Phospho-PKD/PKCµ (Ser744/748) | Cell Signaling Technology | Cat# 2054 RRID:AB_2172539 |
| Rabbit Anti-Phospho-PKD/PKCµ (Ser916) | Cell Signaling Technology | Cat# 2051 RRID:AB_330841 |
| Rabbit Anti-PKD2 (phospho S876), clone (EP1496Y) | Abcam | Cat# ab51251 RRID:AB_882060 |
| Rabbit Anti-Phospho-Akt Substrate (RXXS*/T*) (110B7E) | Cell Signaling Technology | Cat# 9614 RRID:AB_331810 |
| Mouse anti-β-actin | Sigma-Aldrich | Cat# A5441 RRID:AB_476744 |
| Rabbit anti-α-Tubulin | Cell Signaling Technology | Cat# 2144 RRID:AB_2210548 |
| Rabbit anti-Pancreatic Lipase | Abcam | Cat# ab2287737 RRID:AB_2889168 |
| Goat Anti-Villin, clone (C-19) | Santa Cruz Biotechnology | Cat# sc-7672, RRID:AB_2215973 |
| Rabbit Anti-Chromogranin A, clone (H-300) | Santa Cruz Biotechnology | Cat# sc-13090, RRID:AB_2080982 |
| Mouse Anti-E-Cadherin | BD Biosciences | Cat# 610182, RRID:AB_397581 |
| Gpig Anti-Insulin | Abcam | Cat# ab7842 RRID:AB_306130 |
| Goat anti-gpig Alexa Flour^{®} 594 | Thermo Fisher Scientific | Cat# A11076 RRID:AB_141930 |
| Donkey anti-mouse Alexa Flour^{®} 555 | Thermo Fisher Scientific | Cat# A-31570, RRID:AB_2536180 |
| Donkey anti-rabbit Alexa Flour^{®} 647 | Thermo Fisher Scientific | Cat# A-31573, RRID:AB_2536183 |
| Donkey anti-Goat IgG Alexa Flour^{®} 555 | Thermo Fisher Scientific | Cat# A-21447, RRID:AB_2535864 |

| **Bacterial and Virus Strains** | | |
|---|---|---|
| Plasmid: shPkd2. pLKO.1_ MISSION | Sigma-Aldrich | This paper |
| Plasmid: shPkd3. pLKO.1_ MISSION | Sigma-Aldrich | This paper |
| Plasmid: pLKO.1-puro scramble Vector Control_MISSION | Sigma-Aldrich | Cat#: SHC001 |
| Plasmid: pLV[shRNA]-Hygro-U6>Scramble_shRNA | Vector Builder | Cat #: Ecoli (VB010000-0006zba) |
| Plasmid: pLV[shRNA]-Hygro-U6>hPRKD2 | Vector Builder | Cat #: Ecoli (VB201013-1031xdu) |
| Plasmid: psPAX | Addgene | RRID:Addgene_12260 |
| Plasmid: pMD2.G | Addgene | RRID:Addgene_12259 |

| **Chemicals, Peptides, and Recombinant Proteins** | | |
|---|---|---|
| Recombinant human PKD2 protein | Abcam | Cat#: ab60875 |
| Recombinant Human APOA4/Apo-AIV protein | Abcam | Cat#: ab132653 |
| Pkd inhibitor CRT 0066101 | Tocris | Cat#: 4975 |
| Matrigel, Phenol Red-free | Corning | Cat#: 356231 |
| Fluorescein Isothiocyanate Dextran | Sigma-Aldrich | Cat#: 46944 |
| Insulin solution human | Sigma-Aldrich | Cat#: I9278 |
| Triglyceride Reagent | Sigma-Aldrich | Cat#: T2449 |
| Taurocholic acid sodium salt | Sigma-Aldrich | Cat#: T4009 |
| Oleic acid | Sigma-Aldrich | Cat#: 01383 |
| Fatty acids-free BSA | Sigma-Aldrich | Cat#: **A7030** |
| 14C-palmitate | Perkin Elmer | Lot#: 2252435 |
| Fluoromount-G^{™} with DAPI | Thermo Fisher Scientific | Cat#: 00-4959-52 |

| Critical Commercial Assays | | |
|---|---|---|
| Free Glycerol Determination | Sigma-Aldrich | Cat#: F6428 |
| NEFA-reagents | WAKO | Cat#: 999-34691 |
| Ultra Sensitive Mouse Insulin ELISA Kit | Crystal Chem | Cat#: 90080 |

| **Experimental Models: Cell Lines** | | |
|---|---|---|
| Caco-2 cells | ATCC | Cat# HTB-37, RRID:CVCL_0025 |
| HEK293T cells | ATCC | Cat# CRL-3216, RRID:CVCL_0063 |

| **Experimental Models: Organisms/Strains** | | |
|---|---|---|
| Mouse: *Pkd2*^{*S707A*/*S711A*} | The Jackson Laboratory | #017285 |
| Mouse *Pkd2* ^{*Flox*/*Flox*} | Lab. Professor Yamasaki | N/A |
| Mouse: C57BL/6J | The Jackson Laboratory | IMSR Cat# JAX:000664, RRID:IMSR_JAX:0 00664 |
| Mouse: B6.Cg-Tg(Vil1-cre)1000Gum/J | The Jackson Laboratory | Cat# JAX:021504, RRID:IMSR_JAX:0 21504 |

| **Oligonucleotides** | | |
|---|---|---|
| *Pkd2* shRNA lentivirus (Sequence 1) | Sigma-Aldrich | SHCLNG-NM_016457 TRCN0000001948 |
| CCGGCTTCTACGGCCTTTACGACAACTCGA GTTGTCGTAAAGGCCGTAGAAGTTTTT | | |
| *Pkd2* shRNA lentivirus (Sequence 2) | Vector Builder | Cat #: Ecoli (VB201013-1031xdu) |
| TCCAGAACAACACGACCAACAGATACTATA AGGAAATTCCG | | |
| *Pkd3* shRNA lentivirus | Sigma-Aldrich | SHCLNG-NM_005813 TRCN0000195275 |
| CCGGCACTTCATTATGGCTCCTAATCTCGA GATTAGGAGCCATAATGAAGTGTTTTTTG | | |
| Primers for qPCR and genotyping, see Table S1 | | |

| **Software and Algorithms** | | |
|---|---|---|
| Illustrator CS6 | Adobe | https://www.adobe. com/ |
| Image J for Chylomicrons size analysis as in (Wang, Rong et al., 2016a) | NIH | https://imagej.nih.g ov/ij/ |
| LASx | Leica microsystems | https://www.leica-microsystems.com / |

| **Other** | | |
|---|---|---|
| High Fat Research diet | Research Diets | Cat# D12331 |
| Trans-well 1.0 µm pore | Corning | Cat#: 353103 |

### Example 1. Effect of PKD2 inactivation on obesity induced by high-fat diet

Previous studies revealed that PKD1 promotes obesity by blocking energy dissipation in adipocytes (Löffler et al., 2018), while PKD3 promotes hepatic insulin resistance (Mayer et al., 2019). However, the role of PKD2 in regulating glucose and lipid metabolism and in the development of obesity-induced diabetes was not known.

In this study mice with global defective PKD2 enzymatic activity, because of point mutations in serines 707 and 711 to alanines (*Pkd2*^{*ki*/*ki*} mice) were used (Matthews, Navarro et al., 2010a). *Pkd2*^{*ki*/*ki*} mice and corresponding control animals (*Pkd2*^{*wt*/*wt*}) were maintained on a normal chow diet (ND) or high-fat diet (HFD) for 22 weeks after weaning.

Although mice from both groups showed similar weight gain on ND, Pkd2^{ki/ki} mice maintained on HFD gained significantly less weight than the corresponding control group (Fig. 1A). Weight loss in HFD-fed Pkd2^{ki/ki} mice was associated with decreased obesity and decreased adipocyte size (Fig. 1B-E). However, the weight of other organs did not change as a result of PKD2 inactivation (Fig. 1C).

As previous studies indicated that deletion of PKD1 kinase in adipose tissue, promotes the expression of beige adipocytes specific markers (Löffler et al., 2018), expression of *Ucp1, Cidea, Bmp7, Prdm16, Ppara, Pgc1a, Adrb3, Cidec, Myh2, Ckm, Mck, Slc6a8, Slc27a2, Ucp3* and *Myh1* subcutaneous adipose tissue of *Pkd2*^{*ki*/*ki*} and control *Pkd2*^{*wt*/*wt*} mice fed HFD and except *Slc6a8* was tested, which was downregulated in the mice without active PKD2, there were no significant changes in the expression of these genes. Similarly, liver histology and markers of hepatic function, aspartate transaminase (AST) and alanine transaminase (ALT) did not differ between genotypes, while hepatic content of TG was decreased in *Pkd2*^{*ki*/*ki*} mice (Fig 1F). Mice having PKD2 inactivated were also protected from diet-induced glucose intolerance and displayed higher insulin sensitivity when fed HFD (Fig 1G-H). Moreover, in the *Pkd2*^{*ki*/*ki*} mice, we found a small but not significant increase in insulin levels during the glucose-stimulated insulin secretion test. Furthermore, the islet area (relative to pancreas area) is increased in *Pkd2*^{*ki*/*ki*} mice. Inactivation of PKD2 in mice fed HFD also reduced circulating triglyceride and free fatty acid levels (Fig 1I-J). Altogether, these findings indicate that inactivation of PKD2 protects from diet-induced obesity as well as associated hyperglycaemia and hyperlipidemia.

The analyses of metabolic parameters has shown that inactivation of PKD2 does not affect food intake, energy expenditure or voluntary movements of mice fed HFD (Fig 1K-L), suggesting that reduced body weight gain of *Pkd2*^{*ki*/*ki*} upon HFD feeding must be caused by misregulation of other processes.

### Example 2. Effect of PKD2 on the absorption of lipids from food

Since inactivation of PKD2 in mice did not affect food intake and energy expenditure, the effect on this modification on absorption of nutrients was tested. It was observed that feces collected from *Pkd2*^{*ki*/*ki*} mice fed HFD were yellowish in contrast to the excrements derived from control mice which displayed a typical dark-brown color (Fig 2A). Moreover, in contrast to stool from control mice, feces from *Pkd2*^{*ki*/*ki*} mice did not sink in water (Fig 2A). Additionally, *Pkd2*^{*ki*/*ki*} mice fed HFD produced more stool than control animals per week also when extrapolated to food intake (Fig 2B-C). Moreover, HFD feeding was significantly less efficient in promoting body weight gain in mice having inactive PKD2 relative to control animals (Fig 2D). Of note, *Pkd2*^{*ki*/*ki*} fed ND produced the same amount of feces as corresponding control animals and their color did not differ from feces from control animals. This data suggests that PKD2 inactivation dramatically modulates the physicochemical properties of feces of mice fed HFD but not of mice fed ND.

In fact, the analysis of feces content of both genotypes has shown that the feces of Pkd2^{ki/ki} fed with HFD were more energy-dense and contained more lipids than excrements from control mice (Fig 2E-F). Consequently, *Pkd2*^{*ki*/*ki*} mice excreted more energy than corresponding control animals (Fig 2G), which represents a significant decrease in the total metabolizable energy (Fig 2H).

### Example 3. Effect of PKD2 on release of TG from enterocytes

To directly test if PKD2 promotes TG absorption in the intestine, at first it was tested if PKD2 is activated upon ingestion of TG. An increase in PKD2 activity was observed in animals that received olive oil gavage after short-term fasting (Fig 3A). Next, *Pkd2*^{*ki*/*ki*} and *Pkd2*^{*wt*/*wt*} animals were subjected to HFD for one week, and after overnight fasting, a dose of olive oil was administered orally. This evoked over an order of magnitude increase in circulating TG levels in control animals while the same dose of olive oil resulted in significantly lower TG levels in *Pkd2*^{*ki*/*ki*} mice at all the time points (Fig 3B). To test if the decreased levels of TG in *Pkd2*^{*ki*/*ki*} mice results from increased uptake of TG in peripheral organs or decrease absorption in the intestine, the actovity of lipoprotein lipase was blocked by injection of tylaxopol to inhibit TG uptake by the cells from circulation. Importantly, upon ingestion of olive oil, *Pkd2*^{*ki*/*ki*} mice treated with tylaxopol still presented decreased TG levels in the circulation (Fig 3C). These data strongly suggest that PKD2 activity in the intestine promotes TG absorption.

Interestingly, it has been shown that inactivation of PKD2 in the intestine does not affect the expression and activity of PKD1 and PKD3. Western blotting showed that no band corresponding to activated PKD2 (pPKD2 S876) appears in the intestinal extracts isolated from Pkd2^{ki/ki} mice (Fig. 3D). With the use of an antibody recognizing the phosphorylation (activation) of PKD1 (S916) and PKD2 (S876), while the lower band corresponding to PKD1 was not altered by inactivation of PKD2, the upper one was only present in the control mice (Fig. 3D). A similar effect was observed in Caco2 cells with silenced PKD2 (Fig. 3E). Based on these data, the possibility of indirect functioning of other PKD in the intestine as a compensatory mechanism can be excluded.

To investigate the effect of PKD2 on organogenesis in the intestine, organoids derived from crypt stem cells isolated from Pkd2^{ki/ki} and Pkd2^{wt/wt} mice (Zietek, Rath et al., 2015, König, Wells et al., 2016, Mazzawi, Gundersen et al.., 2015). Inactivation of PKD2 did not affect organoid development, its size or cellular composition as demonstrated by staining the markers of intestinal endocrine cells (Chromogranin A) epithelial brush border regulator (Villin) (Fig. 3F). The dextran uptake test also showed that inactivation of PKD2 did not affect gut permeability. The length of the intestine did not change either.

Collectively, these observations indicate that PKD2 promotes intestinal TG uptake by acting directly in enterocytes. This was confirmed by testing in a transwell system containing monolayers of confluent Caco2 cells from the polarized enterocyte lineage. PKd2 silencing in such cells with the help of two independent shRNA sequences significantly reduced the release of TG on the basolateral side of the cell monolayer (Fig. 3G-H). However, the absence of PKD2 had no effect on FFA uptake from the apical side, intracellular lipid retention, or overall cell permeability (Fig. 3G and I). Interestingly, the silencing of PKD3 did not affect the TG uptake. The obtained results indicate that inactivation of PKD2 lowers the ability of enterocytes to resynthesize and resecrete TG, without affecting the intestinal morphology.

### Example 4. The effect of localized deletion of PKD2 in the intestine on lipid absorption

Next, it was tested if PKD2 promotes TG absorption by acting autonomously in the intestine. To this end, mice deficient for PKD2 in the gut were generated by breeding *Pkd2*^{*flox*/*flox*} mice (Ishikawa, Kosako et al., 2016b) with Villin promoter-driven Cre animals (*Pkd2*^{*gutΔ*/*Δ*} mice) (Madison, Dunbar et al., 2002). As determined by qPCR and western blot, *Pkd2*^{*gutΔ*/*Δ*} mice lacked PKD2 protein only in the small intestine. There was lack of activation specific to PKD2 (upper band) (Fig 4A) and marked reduction of total PKD activity was observed (Fig 4B). Similar to the *Pkd2*^{*ki*/*ki*} mice, fasted *Pkd2*^{*gutΔ*/*Δ*} mice after ingestion of olive oil (previously fed HFD for six weeks) shown markedly lower circulating TG levels relative to the corresponding control *Pkd2*^{*flox*/*flox*} mice (Fig 4C). Moreover, in line with the previous results from mice missing PKD2 activity globally, deletion of PKD2 specifically in the intestine resulted in resistance to HFD-induced obesity and body fat accumulation (Fig 4D-E) and improved glucose tolerance (Fig 4F). Moreover, circulating FFA and TG levels were reduced in mice deficient for PKD2 specifically in the intestine (Fig 4G-H). In addition, gut PKD2 deletion did not affect food intake or energy expenditure of mice, as well as intestine length or morphology, and hepatic structure. These results show that deletion of PKD2 specifically in the intestine is sufficient to limit lipid absorption from consumed food and protect against diet-induced obesity as well as glucose intolerance.

The composition of microbiota in the small intestinal is highly dependent on the type of diet consumed and interaction with the cells of the host. On the other hand, microorganisms colonizing the intestine are shown to be of functional relevance in lipid metabolism (Turnbaugh, Ley et al., 2006). To assess intestinal microbiota changes in Pkd2^{gutΔ/Δ} mice and Pkd2^{flox/flox} that were fed HFD for two weeks, small intestinal scrapings as well as cecal content were analyzed using 16S rRNA gene sequencing. Both groups of mice were co-housed to obviate cage effects. Despite the effect of coprophagy on genotype-specific differences, significant changes in the microbial compositions were identified. The alpha diversity of the microbiota species (Shannon Index) was significantly higher in Pkd2^{gutΔ/Δ} mice than in control mice in the duodenum, ileum and jejunum, but not in the cecum (Fig 4I). Analysis of beta diversity showed significant differences in duodenum and jejunum/ileum between the mice genotypes with no significant alterations in the cecum.

Next, specific changes in Bacterial operational taxonomic units (OTUs) in the different gut segments were analyzed. In Duodenum, members of Bacteroides, which are associated with weight loss (Turnbaugh et al., 2006), we identified to be present only in Pkd2^{gutΔ/Δ} mice, but completely absent in control animals (Fig 4J). The deletion of PKD2 in the intestine improves microbiota profile in mice fed HFD.

### Example 5. The effect of PKD2 on chylomicron size

The expression and level of major factors involved in chylomicron formation were analyzed. MOGAT2 and DGAT1 are responsible for TG re-synthesis, however, their expression or the protein levels of MOGAT2 were not affected by PKD2 inactivation (Fig 5A). Similarly, the levels of MTTP, responsible for TG packing into pre-chylomicrons, as well as APOB48 and APOA1, were not affected by PKD2 (Fig 5A). The level of APOA4 protein was markedly elevated in intestine deficient for PKD2 activity or PKD2 inactivation (Fig 5B-C), while the expression of Apoa4 was not affected by PKD2 inactivation. The level of the FA's transporter CD36 was also unchanged in the intestine isolated from Pkd2ki/ki mice (Fig 5A). APOA4 levels were also elevated in serum from these animals while APOB48 and APOA1 levels were not affected by PKD2 inactivation (Fig 5D). Similarly, APOA4 levels were higher in the basolateral fraction secreted by PKD2-depleted Caco2 cells in the transwell system (Fig 5E). APOA4 has been proposed to regulate chylomicron size and to mediate TG secretion by enterocytes (Kohan, Wang et al., 2013). Therefore, the size of chylomicrons in the circulation of mice deficient for PKD2 activity was next determined. Indeed, the size of circulating chylomicrons was markedly reduced in Pkd2^{ki/ki} mice relative to control animals (Fig 5F-G). Since PKD2 inactivation resulted in elevated APOA4 protein but did not affect its expression, PKD2 might regulate APOA4 levels and function by a posttranslational mechanism. Importantly, an in vitro kinase assay revealed that PKD2 phosphorylates APOA4 and that the PKD-specific inhibitor CRT0066101 abrogates PKD2-dependent phosphorylation (Fig 5H). In the presence of APOA4, the phosphorylation of PKD2 was also increased in the kinase assay, suggesting that interaction of PKD2 with this substrate might increase its auto-phosphorylation. Taken together, these results suggest that PKD2 promotes TG packing into chylomicrons presumably by directly phosphorylating and reversing the reported inhibitory role of APOA4 in chylomicrons biogenesis. It is anticipated that high levels of APOA4 in the absence of PKD2 will reduce the size of chylomicrons and lipid transfer.

### Example 6. Inhibition of PKD2 by a small-molecule inhibitor ameliorates diet-induced obesity and diabetes

CRT0066101 inhibitor is an example of a small-molecule compound specifically inhibiting the activity of PKD (Harikumar, Kunnumakkara et al., 2010) and has been proposed as a promising therapeutic agent for the treatment of several types of human diseases (Borges, Perez et al., 2015, Harikumar et al., 2010, Li, Hsu et al., 2018, Sua, Wanga et al., 2019, Thrower, Yuan et al., 2011, Venardos, De Jong et al., 2015, Yuan, Tan et al., 2017). The present inventors found that treatment of Caco2 cells with CRT0066101 reduces TG secretion in a dose dependent manner (Fig. 6A). Interestingly, CRT0066101 did not reduce TG secretion in Caco2 cells deficient in PKD2 (Fig. 6B). The mice were then treated with an oral dose of 10 mg/kg of inhibitor CRT0066101 once a day. At this dose, intestinal PKD activity was attenuated, but no similar decrease was observed in liver or adipose tissue (Fig. 6C). Importantly, treatment of HFD-fed mice with the inhibitor CRT0066101 resulted in greater fecal energy excretion (Fig. 6D), decreased intestinal TG absorption (Fig. 6E), and increased levels of APOA4 in the intestine (Fig. 6F). Long-term treatment of HFD-fed mice with a PKD inhibitor resulted in significantly lower body weight gain compared to the corresponding vehicle (water) treated control animals (Fig. 6G). At the same time, oral administration of CRT0066101 did not affect food consumption, energy expenditure or mobility, as in animals deficient in PKd2. Treatment with CRT0066101 simultaneously improved glucose and insulin tolerance in HFD-fed animals (Figs. 6H-I). Administration of a PKD inhibitor did not affect markers of liver function or accumulation of TG in the liver, but promoted healthier, multilocular brown adipose tissue.

Then it was tested whether inhibition of PKD2 can be helpful in the already established obesity. Therefore, the mice were first fed a high fat diet (HFD) for 7 weeks and then given either CRT0066101 or control solution (water) with continued HFD. The PKD inhibitor significantly decreased weight gain, which was associated with less obesity as well as improved glucose tolerance and insulin sensitivity (Figs. 7A-E). It should be noted that the intestinal permeability was not altered by administration of the inhibitor. These results indicate that inhibition of PKD reduces intestinal TG absorption and thus protects against the development or worsening of obesity and associated diabetes.

It should also be noted here that the activity of PKD2 in the intestine in obese patients correlates positively with triglyceride levels and the percentage of glycated hemoglobin (HbA1c) in the bloodstream (Fig. 7F-H). There is also an inverse correlation between PKD2 activity and HDL levels in these patients, although not at the level of statistical significance (Fig. 7I).

The experiments shown in the examples show that PKD2 promotes the uptake of lipids in the intestine by acting directly in the enterocytes. Correspondingly, the decline in PKD2 function was associated with resistance to obesity and diabetes as well as an improved gut microflora profile. Experiments in human Caco2 cells showed that FFA uptake and re-esterification as well as the retention of TG in enterocytes were not clearly influenced by PKD2. However, PKD2 promoted the packaging of TG into chylomicrons. The lack of PKD2-dependent signaling in mouse enterocytes resulted in a reduction in chylomicrons. At the molecular level, PKD2 regulated the phosphorylation of APOA4, one of the major lipoproteins involved in chylomicron biogenesis. This event may promote lipidation of chylomicrons and prevent their premature release. It has also been shown that inactivation of PKD2 leads to elevated serum APOA4 levels. APOA4 has previously been linked in humans and rodents with a lower range of atherosclerosis and diabetes (Qu, Ko et al., 2019). ApoA4 is mainly synthesized in the intestine and its expression is strongly induced in response to fat consumption (Kohan et al., 2013). Genetic inactivation of Apoa4 in mice results in larger chylomicrons but does not affect TG absorption in the gut (Kohan et al., 2012, Kohan et al., 2013). On the other hand, overexpression of APoA4 in neonatal porcine enterocytes is associated with increased chylomicrons and TG release (Lu et al., 2006, Lu, Yao et al., 2002). Moreover, overexpression of APOA4 in the gut of mice resulted in higher postprandial TG levels in both standard fed and HFD (Aalto-Setälä, Bisgaier et al., 1994). This indicates that a high level of APOA4 does not readily correlate with its function. In the absence of PKD2 dependent signaling, elevated levels of APOA4 protein were observed in the gut and in the serum. However, the expression of Apoa4 did not change in any of the systems tested. Therefore, the level of APOA4 protein appears to be regulated by PKD2 dependent phosphorylation. Another PKD2 dependent function could be the regulation of the retention of APOA4 and chylomicrons in the ER. This model is suggested by studies using an APOA4 mutant carrying an ER retention tag. Expression of this mutant protein in a liver or COS cell line resulted in decreased secretion of APOB-containing lipid particles (Gallagher, Weinberg et al., 2004, Weinberg et al., 2012). Likewise, unphosphorylated APOA4 can be retained at the ER level. Alternatively, intestinal PKD2 can increase lipid absorption, chylomicron formation and release, as well as body weight by regulating the stability/activity of proteins targeting the lipid droplets in the Golgi apparatus (Kim, Kim et al., 2020). It should also be noted that while PKD2 inhibition/deletion did not short-term affect FA uptake on the apical side of Caco2 cells, it resulted in richer fat stools in mice in long-term. This indicates a complex and dynamic role of PKD2 in the regulation of FA uptake and/or release from enterocytes.

In conclusion, the inventors have shown that PKD2 is the main kinase regulating fat absorption in the intestine. PKd2 inhibition, particularly when confined to the gut, has been proven to be an effective approach to the treatment and prevention of obesity, including conditions associated with obesity (e.g., diabetes). PKD2 has also been confirmed to play a similar role in mice, in human cells, and in the human intestine.

### References:

Aalto-Setälä K, Bisgaier CL, Ho A, Kieft KA, Traber MG, Kayden HJ, Ramakrishnan R, Walsh A, Essenburg AD, Breslow JL (1994) Intestinal expression of human apolipoprotein A-IV in transgenic mice fails to influence dietary lipid absorption or feeding behavior. The Journal of clinical investigation 93: 1776-1786
Bedford DC, Kasper LH, Wang R, Chang Y, Green DR, Brindle PKJCm (2011) Disrupting the CH1 domain structure in the acetyltransferases CBP and p300 results in lean mice with increased metabolic control. 14: 219-230
Borges S, Perez EA, Thompson EA, Radisky DC, Geiger XJ, Storz P (2015) Effective Targeting of Estrogen Receptor-Negative Breast Cancers with the Protein Kinase D Inhibitor CRT0066101. Molecular cancer therapeutics 14: 1306-1316
Clara R, Schumacher M, Ramachandran D, Fedele S, Krieger JP, Langhans W, Mansouri A (2017) Metabolic Adaptation of the Small Intestine to Short-and Medium-Term High-Fat Diet Exposure. Journal of cellular physiology 232: 167-175
Edgar RC (2013) UPARSE: highly accurate OTU sequences from microbial amplicon reads. Nature Methods 10: 996-998
El-Merahbi R, Viera JT, Valdes AL, Kolczynska K, Reuter S, Löffler MC, Erk M, Ade CP, Karwen T, Mayer AE (2020) The adrenergic-induced ERK3 pathway drives lipolysis and suppresses energy dissipation. Genes & development 34: 495-510
Fielitz J, Kim M-S, Shelton JM, Qi X, Hill JA, Richardson JA, Bassel-Duby R, Olson EN (2008) Requirement of protein kinase D1 for pathological cardiac remodeling. Proceedings of the National Academy of Sciences 105: 3059-3063
Gallagher JW, Weinberg RB, Shelness GS (2004) apoA-IV tagged with the ER retention signal KDEL perturbs the intracellular trafficking and secretion of apoB. Journal of lipid research 45: 1826-1834
Harikumar KB, Kunnumakkara AB, Ochi N, Tong Z, Deorukhkar A, Sung B, Kelland L, Jamieson S, Sutherland R, Raynham T (2010) A novel small-molecule inhibitor of protein kinase D blocks pancreatic cancer growth in vitro and in vivo. Molecular cancer therapeutics 9: 1136-1146
Henao-Mejia J, Elinav E, Jin C, Hao L, Mehal WZ, Strowig T, Thaiss CA, Kau AL, Eisenbarth SC, Jurczak MJ (2012) Inflammasome-mediated dysbiosis regulates progression of NAFLD and obesity. Nature 482: 179-185
Hernandez Vallejo SJ, Alqub M, Luquet S, Cruciani-Guglielmacci C, Delerive P, Lobaccaro J-M, Kalopissis A-D, Chambaz J, Rousset M, Lacorte J-M (2009) Short-term adaptation of postprandial lipoprotein secretion and intestinal gene expression to a high-fat diet. American Journal of Physiology-Gastrointestinal and Liver Physiology 296: G782-G792
Hesse D, Jaschke A, Chung B, Schürmann A (2013) Trans-Golgi proteins participate in the control of lipid droplet and chylomicron formation. Bioscience reports 33
Hussain MM (2014) Intestinal lipid absorption and lipoprotein formation. Current opinion in lipidology 25: 200
Ishikawa E, Kosako H, Yasuda T, Ohmuraya M, Araki K, Kurosaki T, Saito T, Yamasaki S (2016a) Protein kinase D regulates positive selection of CD4+ thymocytes through phosphorylation of SHP-1. Nature Communications 7: 12756
Ishikawa E, Kosako H, Yasuda T, Ohmuraya M, Araki K, Kurosaki T, Saito T, Yamasaki S (2016b) Protein kinase D regulates positive selection of CD4+ thymocytes through phosphorylation of SHP-1. Nature communications 7: 12756
Ittner A, Block H, Reichel CA, Varjosalo M, Gehart H, Sumara G, Gstaiger M, Krombach F, Zarbock A, Ricci R (2012) Regulation of PTEN activity by p38δ-PKD1 signaling in neutrophils confers inflammatory responses in the lung. Journal of Experimental Medicine 209: 2229-2246
Kim J, Kim H, Noh SH, Jang DG, Park S-Y, Min D, Kim H, Kweon H-S, Kim H, Aum S (2020) Grasp55-/- mice display impaired fat absorption and resistance to high-fat diet-induced obesity. Nature communications 11: 1-17
Kim M-S, Fielitz J, McAnally J, Shelton JM, Lemon DD, McKinsey TA, Richardson JA, Bassel-Duby R, Olson EN (2008) Protein kinase D1 stimulates MEF2 activity in skeletal muscle and enhances muscle performance. Molecular and cellular biology 28: 3600-3609
Kleger A, Loebnitz C, Pusapati GV, Armacki M, Müller M, Tümpel S, Illing A, Hartmann D, Brunner C, Liebau S (2011) Protein kinase D2 is an essential regulator of murine myoblast differentiation. PloS one 6
Klindworth A, Pruesse E, Schweer T, Peplies J, Quast C, Horn M, Glöckner FO (2013) Evaluation of general 16S ribosomal RNA gene PCR primers for classical and next-generation sequencing-based diversity studies. Nucleic acids research 41: e1
Kohan AB, Wang F, Li X, Bradshaw S, Yang Q, Caldwell JL, Bullock TM, Tso P (2012) Apolipoprotein A-IV regulates chylomicron metabolism-mechanism and function. American Journal of Physiology-Gastrointestinal and Liver Physiology 302: G628-G636
Kohan AB, Wang F, Li X, Vandersall AE, Huesman S, Xu M, Yang Q, Lou D, Tso P (2013) Is apolipoprotein A-IV rate limiting in the intestinal transport and absorption of triglyceride? American Journal of Physiology-Gastrointestinal and Liver Physiology 304: G1128-G1135
Kohan AB, Wang F, Lo C-M, Liu M, Tso P (2015) ApoA-IV: current and emerging roles in intestinal lipid metabolism, glucose homeostasis, and satiety. American Journal of Physiology-Gastrointestinal and Liver Physiology 308: G472-G481
Kolczynska K, Loza-Valdes A, Hawro I, Sumara G (2020) Diacylglycerol-evoked activation of PKC and PKD isoforms in regulation of glucose and lipid metabolism: a review. Lipids in Health and Disease 19: 1-15
König J, Wells J, Cani PD, García-Ródenas CL, MacDonald T, Mercenier A, Whyte J, Troost F, Brummer R-J (2016) Human intestinal barrier function in health and disease. Clinical and translational gastroenterology 7: e196
Konopatskaya O, Matthews SA, Harper MT, Gilio K, Cosemans JM, Williams CM, Navarro MN, Carter DA, Heemskerk JW, Leitges M (2011) Protein kinase C mediates platelet secretion and thrombus formation through protein kinase D2. Blood, The Journal of the American Society of Hematology 118: 416-424
Kraus D, Yang Q, Kahn BB (2015) Lipid Extraction from Mouse Feces. Bio Protoc 5
Lagkouvardos I, Fischer S, Kumar N, Clavel T (2017) Rhea: a transparent and modular R pipeline for microbial profiling based on 16S rRNA gene amplicons. PeerJ 5: e2836
Li QQ, Hsu I, Sanford T, Railkar R, Balaji N, Sourbier C, Vocke C, Balaji K, Agarwal PK (2018) Protein kinase D inhibitor CRT0066101 suppresses bladder cancer growth in vitro and xenografts via blockade of the cell cycle at G2/M. Cellular and Molecular Life Sciences 75: 939-963
Löffler MC, Mayer AE, Viera JT, Valdes AL, El-Merahbi R, Ade CP, Karwen T, Schmitz W, Slotta A, Erk M (2018) Protein kinase D1 deletion in adipocytes enhances energy dissipation and protects against adiposity. The EMBO journal 37
Lowe ME (2002) The triglyceride lipases of the pancreas. Journal of lipid research 43: 2007-2016
Lu S, Yao Y, Cheng X, Mitchell S, Leng S, Meng S, Gallagher JW, Shelness GS, Morris GS, Mahan J (2006) Overexpression of apolipoprotein A-IV enhances lipid secretion in IPEC-1 cells by increasing chylomicron size. Journal of Biological Chemistry 281: 3473-3483
Lu S, Yao Y, Meng S, Cheng X, Black DD (2002) Overexpression of apolipoprotein A-IV enhances lipid transport in newborn swine intestinal epithelial cells. Journal of Biological Chemistry 277: 31929-31937
Madison BB, Dunbar L, Qiao XT, Braunstein K, Braunstein E, Gumucio DL (2002) Cis elements of the villin gene control expression in restricted domains of the vertical (crypt) and horizontal (duodenum, cecum) axes of the intestine. Journal of Biological Chemistry 277: 33275-33283
Mansbach CM, Siddiqi S (2016) Control of chylomicron export from the intestine. American Journal of Physiology-Gastrointestinal and Liver Physiology 310: G659-G668
Martinez-Guryn K, Hubert N, Frazier K, Urlass S, Musch MW, Ojeda P, Pierre JF, Miyoshi J, Sontag TJ, Cham CM (2018) Small intestine microbiota regulate host digestive and absorptive adaptive responses to dietary lipids. Cell host & microbe 23: 458-469. e5
Matthews SA, Navarro MN, Sinclair LV, Emslie E, Feijoo-Carnero C, Cantrell DA (2010a) Unique functions for protein kinase D1 and protein kinase D2 in mammalian cells. Biochemical Journal 432: 153-163
Matthews SA, Navarro MN, Sinclair LV, Emslie E, Feijoo-Carnero C, Cantrell DA (2010b) Unique functions for protein kinase D1 and protein kinase D2 in mammalian cells. The Biochemical journal 432: 153-163
Mayer AE, Löffler MC, Valdés AEL, Schmitz W, El-Merahbi R, Viera JT, Erk M, Zhang T, Braun U, Heikenwalder M (2019) The kinase PKD3 provides negative feedback on cholesterol and triglyceride synthesis by suppressing insulin signaling. Science signaling 12: eaav9150
Mayer AE, Valdes AL, Schmitz W, Viera JT, Leitges M, Schlosser A, Sumara G (2020) A phosphoproteomic approach reveals that PKD3 controls phenylalanine and tyrosine metabolism. bioRxiv
Mazzawi T, Gundersen D, Hausken T, El-Salhy M (2015) Increased chromogranin a cell density in the large intestine of patients with irritable bowel syndrome after receiving dietary guidance. Gastroenterology research and practice 2015
McMurdie PJ, Holmes S (2014) Waste Not, Want Not: Why Rarefying Microbiome Data Is Inadmissible. PLOS Computational Biology 10: e1003531
Murtaza M, Khan G, Aftab MF, Afridi SK, Ghaffar S, Ahmed A, Hafizur RM, Waraich RS (2017) Cucurbitacin E reduces obesity and related metabolic dysfunction in mice by targeting JAK-STAT5 signaling pathway. PloS one 12: e0178910
Petit V, Arnould L, Martin P, Monnot M-C, Pineau T, Besnard P, Niot I (2007) Chronic high-fat diet affects intestinal fat absorption and postprandial triglyceride levels in the mouse. Journal of lipid research 48: 278-287
Pilitsi E, Farr OM, Polyzos SA, Perakakis N, Nolen-Doerr E, Papathanasiou A-E, Mantzoros CS (2019) Pharmacotherapy of obesity: available medications and drugs under investigation. Metabolism 92: 170-192
Qin J, Li Y, Cai Z, Li S, Zhu J, Zhang F, Liang S, Zhang W, Guan Y, Shen D (2012) A metagenome-wide association study of gut microbiota in type 2 diabetes. Nature 490: 55-60
Qu J, Ko C-W, Tso P, Bhargava A (2019) Apolipoprotein A-IV: a multifunctional protein involved in protection against atherosclerosis and diabetes. Cells 8: 319
Quast C, Pruesse E, Yilmaz P, Gerken J, Schweer T, Yarza P, Peplies J, Glöckner FO (2013) The SILVA ribosomal RNA gene database project: improved data processing and web-based tools. Nucleic acids research 41: D590-D596
Rozengurt E (2011) Protein kinase D signaling: multiple biological functions in health and disease. Physiology 26: 23-33
Sato T, Vries RG, Snippert HJ, van de Wetering M, Barker N, Stange DE, van Es JH, Abo A, Kujala P, Peters PJ, Clevers H (2009) Single Lgr5 stem cells build crypt-villus structures in vitro without a mesenchymal niche. Nature 459: 262-265
Srinivasan B, Kolli A, Esch M, Abaci H, Shuler M, Hickman J TEER Measurement Techniques for. In Vitro
Sua Q, Wanga LAF, Shia A, Zhanga J, Chenb L (2019) The role and mechanism of CRT0066101 as an effective drug for treatment of triple-negative breast cancer. Cell Physiol Biochem 52: 382-396
Sumara G, Formentini I, Collins S, Sumara I, Windak R, Bodenmiller B, Ramracheya R, Caille D, Jiang H, Platt KA (2009) Regulation of PKD by the MAPK p38δ in insulin secretion and glucose homeostasis. Cell 136: 235-248
Thrower EC, Yuan J, Usmani A, Liu Y, Jones C, Minervini SN, Alexandre M, Pandol SJ, Guha S (2011) A novel protein kinase D inhibitor attenuates early events of experimental pancreatitis in isolated rat acini. American Journal of Physiology-Gastrointestinal and Liver Physiology 300: G120-G129
Trujillo Viera J, El-Merahbi R, Nieswandt B, Stegner D, Sumara G (2016) Phospholipases D1 and D2 Suppress Appetite and Protect against Overweight. PLOS ONE 11: e0157607
Turnbaugh PJ, Ley RE, Mahowald MA, Magrini V, Mardis ER, Gordon JI (2006) An obesity-associated gut microbiome with increased capacity for energy harvest. nature 444: 1027
Uchida A, Whitsitt MC, Eustaquio T, Slipchenko MN, Leary JF, Cheng J-X, Buhman KK (2012) Reduced triglyceride secretion in response to an acute dietary fat challenge in obese compared to lean mice. Frontiers in physiology 3: 26
Venardos K, De Jong KA, Elkamie M, Connor T, McGee SL (2015) The PKD inhibitor CID755673 enhances cardiac function in diabetic db/db mice. PloS one 10
Wang B, Rong X, Duerr MA, Hermanson DJ, Hedde PN, Wong JS, de Aguiar Vallim TQ, Cravatt BF, Gratton E, Ford DAJCm (2016a) Intestinal phospholipid remodeling is required for dietary-lipid uptake and survival on a high-fat diet. 23: 492-504
Wang B, Rong X, Duerr Mark A, Hermanson Daniel J, Hedde Per N, Wong Jinny S, de Aguiar Vallim Thomas Q, Cravatt Benjamin F, Gratton E, Ford David A, Tontonoz P (2016b) Intestinal Phospholipid Remodeling Is Required for Dietary-Lipid Uptake and Survival on a High-Fat Diet. Cell Metab 23: 492-504
Weinberg RB, Gallagher JW, Fabritius MA, Shelness GS (2012) ApoA-IV modulates the secretory trafficking of apoB and the size of triglyceride-rich lipoproteins. Journal of lipid research 53: 736-743
Wilkins LJ, Monga M, Miller AW (2019) Defining dysbiosis for a cluster of chronic diseases. Scientific Reports 9: 1-10
Woting A, Blaut M (2018) Small intestinal permeability and gut-transit time determined with low and high molecular weight fluorescein isothiocyanate-dextrans in C3H mice. Nutrients 10: 685
Xiao Y, Wang C, Chen J-Y, Lu F, Wang J, Hou N, Hu X, Zeng F, Ma D, Sun X (2018) Deficiency of PRKD2 triggers hyperinsulinemia and metabolic disorders. Nature communications 9: 1-11
Xiong J, Zhou M-f, Wang Y-d, Chen L-p, Xu W-f, Wang Y-d, Deng F (2016) Protein kinase d2 protects against acute colitis induced by dextran sulfate sodium in mice. Scientific reports 6: 34079
Xu S, Jay A, Brunaldi K, Huang N, Hamilton JA (2013) CD36 enhances fatty acid uptake by increasing the rate of intracellular esterification but not transport across the plasma membrane. Biochemistry 52: 7254-7261
Yang M, Nickels JT (2015) MOGAT2: a new therapeutic target for metabolic syndrome. Diseases 3: 176-192
Yuan J, Tan T, Geng M, Tan G, Chheda C, Pandol SJ (2017) Novel small molecule inhibitors of protein kinase D suppress NF-kappaB activation and attenuate the severity of rat cerulein pancreatitis. Frontiers in physiology 8: 1014
Zhang S, Liu H, Yin M, Pei X, Hausser A, Ishikawa E, Yamasaki S, Jin ZG (2020) Deletion of Protein Kinase D3 Promotes Liver Fibrosis in Mice. Hepatology
Zhao L, Zhu X, Cong R, Yang X, Zhu Y (2018) The protective effects of danggui-baizhu-tang on high-fat diet-induced obesity in mice by activating thermogenesis. Frontiers in pharmacology 9: 1019
Zietek T, Rath E, Haller D, Daniel H (2015) Intestinal organoids for assessing nutrient transport, sensing and incretin secretion. Scientific reports 5: 1-10

## Claims

1. A PKD protein kinase inhibitor for use in preventing or treating obesity in a subject, preferably also including related conditions such as diabetes, wherein the PKD protein kinase inhibitor is administered orally, **characterized in that** the PKD protein kinase inhibitor is targeted to act locally in the digestive system, especially in the small intestine.

2. The PKD protein kinase inhibitor for use according to claim 1, **characterized in that** the PKD protein kinase inhibitor is a PKD2 inhibitor, in particular a PKD2 specific inhibitor.

3. The PKD protein kinase inhibitor for use according to claim 1 or 2, **characterized in that** the PKD protein kinase inhibitor is administered in a manner that limits the intestinal barrier crossing.

4. The PKD protein kinase inhibitor for use according to any one of claims 1 to 3, **characterized in that** the subject is a high fat food eater, e.g. a subject on a high fat diet.

5. The PKD protein kinase inhibitor for use according to any one of claims 1 to 4, **characterized in that** PKD protein kinase inhibitor is a compound of Formula I 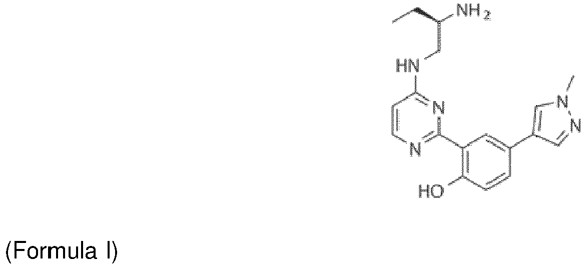 or a pharmaceutically acceptable salt thereof.

6. The PKD protein kinase inhibitor for use according to any one of claims 1 to 5, **characterized in that** the subject is human.

7. The PKD protein kinase inhibitor for use according to claim 6, **characterized in that** the PKD protein kinase inhibitor is administered at a dose 0,1 - 20 mg/kg body weight per day.

8. The PKD protein kinase inhibitor for use according to claim 7, **characterized in that** the PKD protein kinase inhibitor is administered at a dose 5 - 20 mg/kg body weight per day.

9. The PKD protein kinase inhibitor for use according to claim 7, **characterized in that** the PKD protein kinase inhibitor is administered at a dose 5 - 10 mg/kg body weight per day.

10. The PKD protein kinase inhibitor for use according to claim 7, **characterized in that** the PKD protein kinase inhibitor is administered at a dose 0,1 - 5 mg/kg body weight per day.

11. A pharmaceutical composition for use as defined in any one of claims 1-10 comprising a PKD protein kinase inhibitor as defined in any one of claims 1-10, and pharmaceutically acceptable excipients or auxiliary ingredients.

12. The pharmaceutical composition for use according to claim 11, **characterized in that** the composition comprises at least one auxiliary ingredient to reduce absorption in the digestive system, in particular in the intestine.
